(19) Europäisches Patentamt
European Patent Office
Office européen des brevets

(11) Publication number: 0 203 891 B1

(12) # EUROPEAN PATENT SPECIFICATION

(45) Date of publication of patent specification: **15.05.91 Bulletin 91/20**

(51) Int. Cl.$^5$: **C07F 9/547**, C07F 9/60, A61K 31/675

(21) Application number: **86810219.5**

(22) Date of filing: **20.05.86**

(54) **Certain phosphonic acids and derivatives.**

(30) Priority: **24.05.85 US 738102**

(43) Date of publication of application: **03.12.86 Bulletin 86/49**

(45) Publication of the grant of the patent: **15.05.91 Bulletin 91/20**

(84) Designated Contracting States: **AT BE CH DE FR GB IT LI LU NL SE**

(56) References cited:
**GB-A- 2 104 079**
**K. Matoba et al.: Chem. Pharm. Bull. 32, pp 3918-3925(1984)**

(73) Proprietor: **CIBA-GEIGY AG**
**Klybeckstrasse 141**
**CH-4002 Basel (CH)**

(72) Inventor: **Hutchison, Alan J.**
**35 Lynwood Road**
**Verona New Jersey 07044 (US)**
Inventor: **Shaw, Kenneth R.**
**176 Millburn Avenue**
**Millburn New Jersey 07041 (US)**
Inventor: **Schneider, Josef A.**
**99 Main Street**
**Millburn New Jersey 07041 (US)**

EP 0 203 891 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention is concerned with compounds of the formula I,

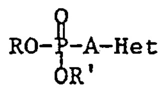 (I),

wherein R and R′ independently represent hydrogen, $C_1$-$C_4$alkyl, benzyl, benzyl substituted on phenyl by halogen, $C_1$-$C_4$alkyl or $C_1$-$C_4$alkoxy, $C_2$-$C_7$alkanoyloxymethyl or $C_2$-$C_7$alkanoyloxymethyl substituted on oxymethyl by $C_1$-$C_4$alkyl or $C_3$-$C_8$cycloalkyl, A denotes $C_1$-$C_4$alkylene and Het represents a 2-$R^1$-pyrrolidinyl, 2-$R^1$-2,5-dihydropyrrolyl, 2-$R^1$-1,2,3,6-tetrahydropyridinyl, 2-$R^1$-1,2,5,6-tetrahydropyridinyl, 2-$R^1$-piperidinyl, 2-$R^1$-tetrahydroquinolinyl, 2-$R^1$-perhydroquinolinyl, 2-$R^1$-carboxy-2,3-dihydroindolyl or 2-$R^1$-perhydroindolyl group which may be additionally N-substituted by $C_1$-$C_4$alkyl, phenyl-$C_1$-$C_4$alkyl, $C_2$-$C_7$alkanoyl, benzoyl, $C_1$-$C_4$alkoxycarbonyl, benzyloxycarbonyl, phenylacetyl, 3-phenylpropionyl or α-amino-$C_2$-$C_4$alkyl, C-substituted in the heterocyclic 5- or 6-ring by $C_1$-$C_4$alkyl or phenyl-$C_1$-$C_4$alkyl and/or, if present, in the benzo or cyclohexano ring by $C_1$-$C_4$ alkyl, $C_1$-$C_4$alkoxy, halo or trifluoromethyl, and with salts thereof.

The instant invention is further concerned with processes for preparing said compounds, with pharmaceutical compositions comprising said compounds, and with their use for the manufacture of pharmaceutical compositions, especially for treating conditions and diseases in mammals responsive to blockade of the N-methyl-D-aspartate (NMDA) sensitive excitatory amino acid receptor.

Phosphono-substituted pyrrolidine- and piperidine-2-carboxylic acid derivative had previously been known from Chem. Pharm. Bull. 32, 3918-3925 (1984) and Chem. Abstr. 104 : 69130a (1986). From GB-A1-2,104,0719 it had also been known that some 2-amino-ω-phosphono-alkanoic acids exhibit NMDA-antagonistic activities. It has now been found that the compounds of the invention are active and useful in mammals as selective antagonists of the N-methyl-D-aspartate (NMDA) sensitive excitatory amino acid receptor. The compounds of the invention are therefore useful, administered alone or in combination to mammals, for the treatment of disorders responsive to said blockade of the NMDA receptor, comprising e.g. cerebral ischaemia, muscular spasms (spasticity), convulsive disorders (epilepsy) and anxiety.

One preferred aspect of the invention relates to the phosphonic acid derivatives of the formula II

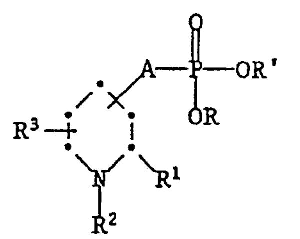 (II)

or a compound of formula II with a double bond present between C-3 and C-4 or between C-4 and C-5 of the piperidinyl ring, in which the phosphono bearing chain is attached at the 3-, 4-, or 5-position of the piperidinyl or tetrahydropyridinyl ring, and wherein R and R′ independently represent hydrogen, $C_1$-$C_4$alkyl, benzyl, $C_2$-$C_4$alkanoyloxymethyl or $C_2$-$C_2$alkanoyloxymethyl substituted on oxymethyl by $C_1$-$C_4$alkyl, cyclohexyl or cyclopentyl, $R^1$ represents carboxy, carbamoyl, N-mono- or N,N-di-$C_1$-$C_4$alkylcarbamoyl or $C_1$-$C_4$alkoxycarbonyl, $R^2$ and $R^3$ represent hydrogen or $C_1$-$C_4$alkyl and A represents $C_1$-$C_4$alkylene ; and salts thereof.

Preferred are the compounds of formula II, wherein R and R′ independently represent hydrogen, $C_2$-$C_4$alkanoyloxymethyl or $C_2$-$C_4$-alkanoyloxymethyl substituted on oxymethyl by $C_1$-$C_4$alkyl, $R^1$ represents carboxy, carbamoyl or $C_1$-$C_4$alkoxycarbonyl, $R^2$ and $R^3$ represent hydrogen and A is at the 4-position and represents $C_1$-$C_4$alkylene ; and pharmaceutically acceptable salts thereof.

Particularly preferred are the compounds of formula III

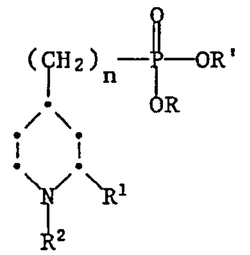

(III)

wherein n represents the integer 1, 2 or 3 ; R and R′ independently represent hydrogen, $C_1$-$C_4$alkyl, benzyl, $C_2$-$C_4$alkanoyloxymethyl or $C_2$-$C_4$-alkanoyloxymethyl substituted on oxymethyl by $C_1$-$C_4$alkyl, $R^1$ represents carboxy, $C_1$-$C_4$alkoxycarbonyl or carbamoyl and $R^2$ represents hydrogen, $C_1$-$C_4$alkyl, $C_2$-$C_4$alkanoyl or benzoyl; and pharmaceutically acceptable salts of said compounds having a salt-forming functional grouping.

Most preferred are the compounds of formula III wherein the 2- and 4-substituents are cis to each other.

Further preferred are the said compounds of formula III wherein n represents the integer 1, 2 or 3 ; R and R′ both represent hydrogen or $C_2$-$C_4$alkanoyloxymethyl ; or one of R and R′ represents hydrogen and the other of R and R′ represents $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, $R^1$ represents carboxy or $C_1$-$C_4$alkoxycarbonyl and $R^2$ represents hydrogen ; and pharmaceutically acceptable salts thereof.

A preferred embodiment relates to the compounds, preferably cis, of formula III wherein n represents the integer 1, 2 or 3 ; R and R′ represent hydrogen ; $R^1$ represents carboxy or $C_1$-$C_4$alkoxycarbonyl ;

$R^2$ represents hydrogen ; and pharmaceutically acceptable salts thereof.

Another aspect of the invention relates to the phosphonic acid derivatives of the formula IV

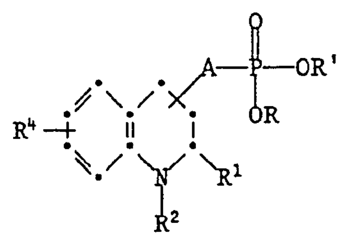

(IV)

or perhydro derivatives thereof, wherein R and R′ independently represent hydrogen, $C_1$-$C_4$alkyl, benzyl, benzyl substituted on phenyl by halogen, $C_1$-$C_4$alkyl or $C_1$-$C_4$alkoxy ; $C_2$-$C_4$alkanoyloxymethyl or $C_2$-$C_4$alkanoyloxymethyl substituted on oxymethyl by $C_1$-$C_4$alkyl or cycloalkyl, $R^1$ represents carboxy, $C_1$-$C_4$alkoxycarbonyl, carbamoyl or N-mono- or N,N-di-$C_1$-$C_4$alkylcarbamyl, $R^2$ represents hydrogen, $C_1$-$C_4$alkyl, aryl-$C_1$-$C_4$alkyl, $C_2$-$C_7$alkanoyl, benzoyl, $C_1$-$C_4$alkoxycarbonyl, phenylacetyl, 3-phenylpropionyl or $\alpha$-amino-$C_2$-$C_4$alkanoyl, $R^4$ represents hydrogen, lower alkyl, lower alkoxy, halogen or trifluoromethyl and A represents $C_1$-$C_4$-alkylene ; and salts thereof.

Preferred are the compounds of formula V

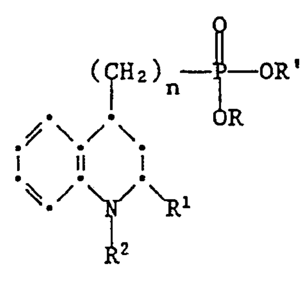

(V)

or the perhydroquinoline derivatives thereof wherein n represents the integer 1, 2 or 3 ; R and R′ independently represent hydrogen, $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, $R^1$ represents carboxy, $C_1$-$C_4$alkoxycarbonyl or carbamoyl, $R^2$ represents hydrogen, $C_1$-$C_4$alkyl, $C_2$-$C_4$alkanoyl, benzoyl or benzoyl substituted by $C_1$-$C_4$alkyl, by $C_1$-$C_4$alkoxy, by halogen or by trifluoromethyl ; and pharmaceutically acceptable salts of said compounds having a salt-forming functional grouping.

Most preferred are the compounds of formula V wherein the 2- and 4- substituents are cis to each other.

A preferred embodiment relates to the compounds, preferably cis, of formula V and the perhydroquinoline derivatives thereof wherein n represents the integer 1, 2 or 3 ; R and R′ represent hydrogen ; $R^1$ represents carboxy or $C_1$-$C_4$alkoxycarbonyl, di-lower alkylamino straight chain and $R^2$ represents hydrogen ; and pharmaceutically acceptable salts thereof.

A further aspect of the invention relates to the phosphonic acid derivatives of the formula VI

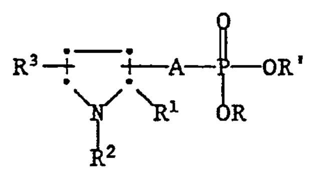

(VI)

or a compound of formula VI with a double bond present between C-3 and C-4 of the pyrrolidinyl ring, wherein R and R′ independently represent hydrogen, $C_1$-$C_4$alkyl, benzyl, benzyl substituted on phenyl by halogen, $C_1$-$C_4$alkyl or $C_1$-$C_4$alkoxy ; $C_2$-$C_4$alkanoyloxymethyl or $C_2$-$C_4$alkanoyloxymethyl substituted on oxymethyl by $C_1$-$C_4$alkyl or cycloalkyl, $R^1$ represents carboxy, $C_1$-$C_4$alkoxycarbonyl, carbamoyl or N-mono- or N,N-di-$C_1$-$C_4$-alkylcarbamoyl, $R^2$ represents hydrogen, $C_1$-$C_4$alkyl, $C_2$-$C_7$alkanoyl, benzoyl, $C_1$-$C_4$alkoxycarbonyl, phenylacetyl, 3-phenylpropionyl or $\alpha$-amino-$C_2$-$C_4$alkanoyl, $R^3$ represents hydrogen, $C_1$-$C_4$alkyl or aryl-$C_1$-$C_4$alkyl and A represents $C_1$-$C_4$alkylene ; and salts thereof.

Preferred are the compounds of formula VI wherein the phosphono bearing group is attached at the 3- or 4-position, R and R′ independently represent hydrogen or $C_2$-$C_4$alkanoyloxymethyl, $R^1$ represents carboxy, $C_1$-$C_4$alkoxycarbonyl or carbamoyl, $R^2$ and $R^3$ represent hydrogen and A represents $C_1$-$C_3$alkylene ; and pharmaceutically acceptable salts thereof.

The general definitions used herein have the following meaning in the context of the invention.

The term "lower", when referred to hereinafter in connection with organic groups, radicals or compounds respectively, defines such with up to and including 7, preferably up to and including 4 carbon atoms.

$C_1$-$C_4$Alkyl preferably represents for example ethyl, propyl, butyl or advantageously methyl.

$C_1$-$C_4$Alkoxy preferably represents for example ethoxy, propoxy or advantageously methoxy.

$C_2$-$C_7$Alkanoyl preferably represents advantageously acetyl, propionyl, n-butyryl, isobutyryl or pivaloyl.

$C_2$-$C_7$Alkanoyloxy represents advantageously acetoxy, propionyloxy, n- or i-butyryloxy or pivaloyloxy.

$C_3$-$C_8$Cycloalkyl contains preferably represents e.g. cyclohexyl or cyclopentyl.

Halogen is preferably fluorine and chlorine, but may also represent bromine or iodine.

Phenyl-$C_1$-$C_4$alkyl preferably represents benzyl or 2-phenylethyl.

$C_1$-$C_4$Alkoxycarbonyl contains 1 to 4 carbon atoms in the alkoxy portion and represents, for example, methoxycarbonyl, propoxycarbonyl, isopropoxycarbonyl or advantageously ethoxycarbonyl.

N-Mono-$C_1$-$C_4$alkylcarbamoyl is, for example, N-methylcarbamoyl, N-propylcarbamoyl or advantageously N-ethylcarbamoyl.

N,N-Di-$C_1$-$C_4$alkylcarbamoyl represents, for example, N-N-dimethyl-carbamoyl, N-ethyl-N-methylcarbamoyl and advantageously N,N-diethyl-carbamoyl.

Salts of the compounds of the invention are preferably pharmaceutically acceptable salts, on the one hand metal or ammonium salts of the compounds of the invention having a free phosphonic or carboxy group, more particularly alkali or alkaline earth metal salts, e.g. the sodium, potassium, magnesium or calcium salt ; or advantageously crystallizing ammonium salts derived from ammonia or organic amines, such as methylamine, diethylamine, triethylamine, dicyclohexylamine, triethanolamine, ethylenediamine, tris-(hydroxymethyl)aminomethane or benzyltrimethylammonium hydroxide. On the other hand the compounds of the invention which are basic amines form acid addition salts of preferably pharmaceutically acceptable inorganic or organic acids, such as of strong mineral acids, for example hydrohalic, e.g. hydrochloric or hydrobromic acid ; sulfuric, phosphoric or nitric acid ; aliphatic or aromatic carboxylic or sulfonic acids, e.g. acetic, propionic, succinic, glycolic, lactic, malic, tartaric, gluconic, citric, ascorbic, maleic, fumaric, pyruvic, pamoic, nicotinic, methanesulfonic, ethanesulfonic, hydroxyethanesulfonic, benzenesulfonic, p-toluenesulfonic or naphthalenesulfonic acid.

For isolation or purification purposes, salts may be obtained which might not be useful for pharmaceutical purposes. However, only pharmaceutically acceptable salts are used for therapeutic purposes and these salts, therefore, are preferred.

The compounds of the invention exhibit valuable pharmacological properties, e.g. by selectively blocking the N-methyl-D-aspartate sensitive excitatory aminoacid receptors in mammals. The compounds are thus useful for treating diseases responsive to excitatory amino acid blockade in mammals, comprising e.g. nervous

system disorders, particularly convulsive disorders (epilepsy) and anxiety.

These effects are demonstrable in in vitro tests or in vivo animal tests using advantageously mammals or tissues or enzyme preparations thereof, e.g. mice, rats, or monkeys. Said compounds can be administered to them enterally or parenterally, advantageously orally or transdermally, or subcutaneously, intravenously or intraperitoneally, for example, within gelatin capsules, or in the form of aqueous suspensions or solutions, respectively. The applied in vivo dosage may range between about 0.01 to 100 mg/kg, preferably between about 0.05 and 50 mg/kg, advantageously between about 0.1 and 10 mg/kg. Said compounds can be applied in vitro in the form of e.g. aqueous solutions and the dosage may range between about $10^{-4}$ molar and $10^{-8}$ molar concentrations.

The inhibitory effect on the NMDA-type excitatory amino acid receptors is determined in vitro by measuring the inhibition of the NMDA-evoked $^3$H-acetylcholine ($^3$H-ACh) release from corpus striatum tissue of rat brain, according to J. Lehmann and B. Scatton, Brain Research 252, 77-89 (1982) and Nature 297, 422-424 (1982).

Antagonists of NMDA-type excitatory amino acid receptors competitively antagonize NMDA-evoked $^3$H-acetylcholine ($^3$H-ACh) release from corpus striatum tissue of the brain.

The inhibition of the NMDA-evoked $^3$H-acetylcholine ($^3$H-ACh) release from rat striatal tissue slices by a compound of the invention is expressed as % of release of $^3$H-ACh in response to stimulation with 50 μM NMDA compared to control. Tests are two-tailed with a minimum of n = 4 in each group. $IC_{50}$ values represent the concentration of test compound required to inhibit the NMDA-increased $^3$H-ACh release by 50%.

Illustrative compounds of the invention have $IC_{50}$-values as low as about $8 \times 10^{-6}$M in the in vitro NMDA-evoked $^3$H-ACh release test.

The inhibitory effect on the NMDA-type excitatory amino acid receptors is demonstrated in vivo by inhibition of NMDA-induced convulsions in the mouse.

Illustrative compounds of the invention prevent NMDA-induced convulsions in the mouse at doses as low as about 2.3 mg/kg i.p.

Further indicative of the anticonvulsant activity, compounds of the invention are effective in preventing audiogenic-induced seizures in DBA/2 mice [Chapman et al., Arzneim. -Forsch.34 ; 1261, (1984)].

The effect is determined as follows : Forty-five minutes following compound or vehicle administration, mice are placed individually in a soundproof chamber. After a 30 second accommodation period, the mice are exposed to a sound stimulation of 110 dB for 1 minute or until the appearance of a tonic-clonic seizure. Control seizures consist of an initial wild running phase. The prevention of wild running is indicative of an anticonvulsant effect.

Test compounds in either distilled water solution or in a 3% (w/v) colloidal cornstarch suspension containing 5% (w/v) polyethylene-glycol 400 and 0.34% (w/v) Tween 80®, are administered by oral intubation or intraperitoneally in a volume of 10 ml/kg of body weight.

Illustrative compounds of the invention are effective in the audiogenicinduced seizure model in mice with an $ED_{50}$ as low as about 1.8 mg/kg on i.p. administration.

Indicative of anxiolytic activity, compounds of the invention are effective in the Cook/Davidson conflict model [Psychophamacologia 15, 159-168 (1969)].

The aforementioned advantageous properties render the compounds of the invention useful as antagonists of the N-methyl-D-aspartate excitatory amino acid receptor in mammals and for the treatment of conditions responsive thereto, such as anxiety and convulsive disorders.

The compounds of the invention, i.e. the compounds cited hereinabove, may be prepared by conventional processes, e.g. by

a) reducing a compound of the formula VII

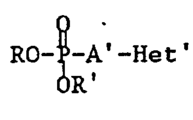

or a functional phosphonic acid derivative thereof as defined above, said compound of formula VII being otherwise identical to a compound of formula I but having one or more double bonds present in the five- or six-membered heterocyclic ring Het′ and/or within A′ which represents A or lower alkenylene or lower alkanylidene with the double bond attached to the heterocyclic ring ; or

b) condensing a compound of the formula VIII

Het-A-X (VIII),

wherein Het and A are as defined for formula I and X represents reactive esterified hydroxy, with a compound capable of introducing the phosphonic acid moiety, having one of formulae IX or X,

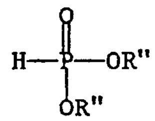 (IX) $P(R''')_3$ (X)

wherein R″ represents lower alkyl and R‴ represents halogen or lower alkoxy and, if required, converting the resulting phosphonic acid derivative to the phosphonic acid or other ester derivative thereof ; or

c) converting to $R^1$ a substituent other than $R^1$ at position 2 of the heterocyclic ring in a compound otherwise identical to a compound of the invention ; and carrying out the said processes while, if necessary, temporarily protecting any interfering reactive group(s) in these processes, and then liberating the resulting compound of the invention ;

and, if desired, converting a resulting compound of the invention into another compound of the invention, and/or, if desired, converting a resulting free compound into a salt or a resulting salt into the free compound or into another salt ; and/or separating a mixture of isomers or racemates obtained into the single isomers or racemates ; and/or, if desired, resolving a racemate obtained into the optical antipodes.

Reactive esterified hydroxy, in any of the herein mentioned processes, e.g. X in a compound of formula VIII, is esterified by a strong acid, especially hydrohalic, e.g. hydrochloric, hydrobromic or hydroiodic acid, or sulphuric acid, or by a strong organic acid, especially a strong organic sulfonic acid, such as an aliphatic or aromatic sulfonic acid, for example methanesulfonic acid, 4-methylphenylsulfonic acid or 4-bromophenylsulfonic acid. Said reactive esterified hydroxy is especially halo, for example chloro, bromo or iodo, or aliphatically or aromatically substituted sulfonyloxy, for example methanesulfonyloxy, phenylsulfonyloxy or 4-methylphenylsulfonyloxy (tosyloxy).

In starting compounds and intermediates therefor which are converted to the compounds of the invention in a manner described herein, functional groups present, such as carboxy, amino (including ring NH) and hydroxy groups, are optionally protected by conventional protecting groups that are common in preparative organic chemistry. Protected carboxy, amino and hydroxy groups are those that can be converted under mild conditions into free carboxy, amino and hydroxy groups without the molecular framework being destroyed or other undesired side reactions taking place.

The purpose of introducing protecting groups is to protect the functional groups from undesired reactions with reaction components and under the conditions used for carrying out a desired chemical transformation. The need and choice of protecting groups for a particular reaction is known to those skilled in the art and depends on the nature of the functional group to be protected (carboxy group, amino group, etc.), the structure and stability of the molecule of which the substituent is a part, and the reaction conditions.

Well-known protecting groups that meet these conditions and their introduction and removal are described, for example, in J.F.W. McOmie, "Protective Groups in Organic Chemistry", Plenum Press, London, New York 1973, T.W. Greene, "Protective Groups in Organic Synthesis", Wiley, New York 1981, and also in "The Peptides", Vol. I, Schroeder and Luebke, Academic Press, London, New York 1965, as well as in Houben-Weyl, "Methoden der Organischen Chemie", Vol. 15/I, Georg Thieme Verlag, Stuttgart, 1974.

The preparation of the compounds of the invention according to process (a) relating to reduction of a compound of formula VII is e.g. carried out by methods known in the art for the reduction of pyrrole, pyridine, indole and quinoline rings. For example, the reduction of the pyridine or quinoline ring is advantageously carried out with an organometallic reducing agent or by catalytic hydrogenation e.g. in the presence of platinum oxide and an acidic solvent such as acetic acid to give corresponding piperidines, 1,2,3,4-tetrahydroquinolines or perhydroquinolines of the invention, i.e. of formula II or IV and derivatives thereof. Quaternary quinolinium and pyridinium compounds, e.g. in which $R^2$ is lower alkyl or aryl-lower alkyl, may be similarly reduced.

These starting compounds of formula VII, wherein Het′ has one or more double bonds, e.g. the quinoline and pyridine derivatives, wherein Het denotes an optionally substituted $R^1$-pyridyl or $R^1$-quinolinyl group and A is $C_1$-$C_4$alkylene, are prepared, e.g. by first condensing a compound of the formula Het′-A-X (VIIa) e.g. in form of a halide, such as a chloride or bromide, with a diester of formula IX in the presence of a strong base, e.g. as described in Chemical Abstracts 61, 10703, or with a tri-(lower)alkyl phosphite of formula X to give the corresponding $R^3$-substituted-(3-, 4- or 5-pyridinyl)-A-P(= O)(O-lower alkyl)$_2$ or $R^4$-substituted-(3- or 4-quinolinyl)-A-P(= O)(O-lower alkyl)$_2$ compound in which A, $R^3$ and $R^4$ are as defined hereinabove.

Subsequent treatment with e.g. a peracid, such as m-chloroperbenzoic acid gives the corresponding pyridine-N-oxides or quinoline-N-oxides. Condensation with a reactive cyanide, e.g. a trialkylsilyl cyanide such as

trimethylsilyl cyanide, preferably under basic conditions, e.g. in the presence of triethylamine, gives the corresponding 2-cyanopyridine or 2-cyanoquinoline derivatives. The cyano groups in the 2-cyanopyridine and 2-cyanoquinoline derivatives are then converted, by methods known in the art, to the 2-$R^1$-substituted pyridine and quinoline derivatives as defined hereinabove.

The preparation of the compounds of formula I and derivatives from compounds of formula VII, wherein A' represents lower alkenylene or lower alkanylidene with the double bond attached to the heterocyclic ring, according to process (a) is carried out by e.g. catalytic hydrogenation.

These starting materials of formula VII may be prepared, e.g. by reacting an aldehyde or ketone (including oxo derivatized heterocyclic five- or six-membered rings) with e.g. a tetra-lower alkyl ester of methylenedi-phosphonic acid under basic conditions, thereby adding the phosphono-methylidene grouping to obtain a compound of formula VII wherein A' represents trivalent methylidene with the double bond attached to the heterocyclic ring.

The condensation according to process (b) of a compound of formula VIII with a compound of formula X, e.g. triethyl phosphite, is carried out, e.g. by heating in an inert solvent, and under conditions known in the art for a Michaelis-Arbuzov reaction according to Angew. Chem. Int. Ed. 16, 477 (1977) and Chem. Rev. 81, 415 (1981). Similarly, condensation with e.g. phosphorus trichloride and subsequent hydrolysis gives a compound of formula I.

The condensation according to process (b) of a compound of formula VIII with a compound of formula IX, e.g. diethylphosphonate (diethyl phosphite), is carried out e.g. in a strong basic medium, for instance in the presence of an alkali metal, e.g. sodium, an alkali metal hydride, e.g. sodium hybride, an alkali metal alkoxide, e.g. potassium t-butoxide, in an inert solvent e.g. toluene or dimethylformamide.

The starting materials of formula VIII are either known in the art or are prepared by methods known in the art, e.g. by reduction of the corresponding pyridinyl, indolyl and quinolinyl compounds.

Interconversions according to process (c) are carried out by methods well-known in the art.

Groups convertible into $R^1$ are, for example, carboxy groups in form of anhydrides or acid halides, cyano, amidino groups, including cyclic amidino groups such as 5-tetrazolyl, iminoether groups, including cyclic iminoether groups, e.g. dihydro-2-oxazolinyl or dihydro-2-oxazolinyl groups substituted by lower alkyl, and also hydroxymethyl, etherified hydroxymethyl, lower alkanoyloxymethyl, trialkoxymethyl, acetyl, trihalo-acetyl, halomethyl, carboxycarbonyl (COCOOH), formyl (CHO), di(lower)alkoxymethyl, alkylenedioxymethyl or vinyl.

Certain terms used in the processes have the meanings as defined below.

Lower alkenylene preferably contains 2 to 4 carbon atoms and represents for example ethenylene, propenylene or butenylene, e.g. 1,2-ethenylene, 1,3-propenylene or 1,4-but-1-enylene.

Lower alkanylidene preferably contains 1 to 4 carbon atoms and represents for example ethanylidene, propanylidene, butanylidene, or, preferably methylidene.

Trialkoxymethyl represents preferably tri(lower alkoxy)methyl, particularly triethoxy- or trimethoxymethyl.

Etherified hydroxymethyl represents preferabyl lower alkoxymethyl, lower alkoxyalkoxymethyl, e.g. methoxymethoxymethyl or 2-oxa- or 2-thiacyclo-alkoxymethyl, particularly 2-tetrahydropyranyloxymethyl.

Halomethyl represents especially chloromethyl but may also be bromomethyl or iodomethyl.

An alkali metal represents preferably lithium but may also be potassium or sodium.

Groups convertible into $R^1$ representing carboxy include esterified and amidated carboxy and such are not limited to esterified and amidated carboxy as defined herein for $R^1$. Conversion to carboxy is generally accomplished by solvolysis, with acid or base.

Benzyloxycarbonyl or nitrobenzyloxycarbonyl may be converted into carboxy by catalytic hydrogenation, the latter also with chemical reducing agents, e.g. sodium dithionite or with zinc and a carboxylic acid. In addition, tert-butyloxycarbonyl may also be cleaved with trifluoroacetic acid.

Acetyl may be oxidatively cleaved to carboxy by conversion first to trihaloacetyl, e.g. tribromo or triiodoacetyl, by treatment e.g. with sodium hypobromite, followed by cleavage with e.g. an aqueous base, e.g. sodium hydroxide. Formyl, di(lower)-alkoxymethyl or alkylenedioxymethyl (formyl protected in the form of an acetal), e.g. the dimethyl acetal, are oxidized with e.g. silver nitrate, pyridinium dichromate or ozone to carboxy.

Vinyl may be converted to carboxy by ozonolysis to formyl, which is in turn oxidized to carboxy.

Hydrolysis of trialkoxymethyl to carboxy is advantageously carried out with inorganic acids, e.g. hydrohalic or sulfuric acid. Hydrolysis of etherified hydroxymethyl to hydroxymethyl is preferably carried out with solutions of inorganic acids, e.g. a hydrohalic acid. Hydroxymethyl is in turn oxidized to carboxy with an oxidizing agent, e.g. pyridinium dichromate.

Halomethyl may also be converted to the corresponding carboxaldehydes with e.g. dimethylsulfoxide in the presence of triethylamine and silver tetrafluoroborate, or with chromium trioxide and pyridine in methylene chloride.

The conversion of cyano to $C_1$-$C_4$alkoxycarbonyl is advantageously carried out by treatment first with a

lower alkanol, e.g. anhydrous ethanol, in the presence of a strong acid, e.g. hydrochloric acid preferably at reflux temperature, followed by hydrolysis with water.

Furthermore, the conversion of cyano to carbamoyl is preferably carried out by treatment with an alkali metal hydroxide, e.g. dilute sodium hydroxide, and hydrogen peroxide, preferably at room temperature.

$C_1$-$C_4$Alkoxycarbonyl may be amidized with ammonia or a N-mono- or N,N-di-$C_1$-$C_4$alkylamine e.g. methylamine or dimethylamine in an inert solvent, e.g. a lower alkanol, such as butanol, to unsubstituted, N-mono- or N,N-di-$C_1$-$C_4$alkylcarbamoyl.

The compounds of the invention may thus also be converted to other compounds of the invention by e.g. functional group transformations well-known in the art.

For example, conversion of carboxylic acid esters and amides to carboxylic acids is advantageously carried out by hydrolysis with inorganic acids such as a hydrohalic or sulfuric acid or with aqueous alkalies, preferably alkali metal hydroxides such as lithium or sodium hydroxide.

Free carboxylic acids may be esterified with $C_1$-$C_4$alkanols, such as ethanol, in the presence of a strong acid, e.g. sulfuric acid, or with diazo $C_1$-$C_4$alkanes, e.g. diazomethane, in a solvent such as ethyl ether, advantageously at room temperature, to give the corresponding lower alkyl esters.

Furthermore, the free carboxylic acids may be converted via treatment of a reactive intermediate thereof, e.g. an acyl halide such as the acid chloride, or a mixed anhydride, e.g. such derived from a lower alkyl halocarbonate such as ethyl chloroformate, with ammonia or an N-mono- or N,N-di-$C_1$-$C_4$alkylamine, in an inert solvent such as methylene chloride, preferably in the presence of a basic catalyst such as pyridine, to compounds wherein $R^1$ represents unsubstituted, N-mono or N,N-di-$C_1$-$C_4$-alkylcarbamoyl.

Phosphonic acid esters are converted to the corresponding phosphonic acids by treatment with acid, such as aqueous hydrochloric acid or hydrobromic acid in glacial acetic acid, or with bromotrimethylsilane according to J. Chem. Soc. Chem. Comm. 1979, 739. Benzyl esters may be converted to the acids by hydrogenolysis.

Phosphonic acids are converted to esters, e.g. $C_1$-$C_4$alkyl or $C_2$-$C_7$-alkanoyloxymethyl esters, e.g. by condensation with a $C_1$-$C_4$alkyl halide or $C_2$-$C_7$alkanoyloxymethylhalide preferably in a basic non-aqueous medium, such as in the presence of triethylamine.

The compounds of the invention wherein the heterocylic ring represents optionally substituted tetrahydroquinolinyl, dihydroindolyl or dihydropyrrolyl) and derivatives thereof are converted to the corresponding perhydroquinolinyl, perhydroindolyl or pyrrolidinyl compounds, respectively, e.g. by catalytic hydrogenation.

The above-mentioned reactions are carried out according to standard methods, in the presence or absence of diluents, preferably such that are inert to the reagents and are solvents thereof, of catalysts, of condensing or said other agents respectively and/or in inert atmospheres, at low temperatures, room temperature or elevated temperatures, preferably at the boiling point of the solvents used, and at atmospheric or super-atmospheric pressure. The preferred solvents, catalysts and reaction conditions are set forth in the appended illustrative examples.

The invention further includes any variant of the present processes, in which an intermediate product obtainable at any stage thereof is used as starting material and the remaining steps are carried out, or the process is discontinued at any stage thereof, or in which the starting materials are formed under the reaction conditions, or in which the reaction components are used in the form of their salts or optically pure antipodes.

Mainly those starting materials should be used in said reactions, that lead to the formation of those compounds indicated above as being especially preferred.

The invention also relates to any novel starting materials and processes for their manufacture.

Depending on the choice of starting materials and methods, the new compounds may be in the form of one of the possible isomers or mixtures thereof, for example, depending on the number of asymmetrical carbon atoms, as pure optical isomers, such as antipodes, or as mixtures of optical isomers such as racemates or as mixtures of diastereoisomers or of geometric isomers. The aforesaid possible isomers or mixtures thereof are within the purview of this invention ; certain particular isomers are preferred as indicated above.

Any resulting mixtures of diastereoisomers, mixtures of racemates can be separated on the basis of the physicochemical differences of the constituents, in known manner, into the pure isomers, diastereoisomers, racemates, or geometric isomers, for example by chromatography and/or fractional crystallization.

Any resulting racemates can be resolved into the optical antipodes by known methods, for example by e.g. reacting an acidic end product with an optically active base that forms salts with the racemic acid, and separating the salts obtained in this manner, for example by fractional crystallization, into the diastereoisomeric salts from which the optically active free carboxylic or phosphonic acid antipodes can be liberated on acidification. The basic racemic products can likewise be resolved into the optical antipodes, e.g. by separation of the diastereo-isomeric salts thereof, with an optically active acid, and liberating the optically active basic compound by treatment with a standard base. Racemic products of the invention can thus be resolved into their optical antipodes, e.g., by the fractional crystallization of d- or $\ell$-(tartrates, mandelates, camphorsulfonates) or of d- or-

ℓ(α-methylbenzylamine, cinchonidine, cinchonine, quinine, quinidine, ephedrine, dehydroabiethylamine, brucine or strychnine) salts. The acidic compounds of the invention can also be resolved by separating diastereomeric ester or amide derivatives prepared from an optically active alcohol or amine, and regenerating the resolved optically active compound. Advantageously, the more active of the two antipodes is isolated.

Finally the compounds of the invention are either obtained in the free form, or as a salt thereof. Any resulting base can be converted into a corresponding acid addition salt, preferably with the use of a therapeutically useful acid or anion exchange preparation, or resulting salts can be converted into the corresponding free bases, for example, with the use of a stronger base, such as a metal or ammonium hydroxide or a basic salt, e.g. an alkali metal hydroxide or carbonate, or a cation exchange preparation, or an alkylene oxide such as propylene oxide. A compound of the invention with a free carboxylic or phosphonic acid group can thus also be converted into the corresponding metal or ammonium salts. These or other salts, for example, the pricrates, can also be used for purification of the bases obtained ; the bases are converted into salts, the salts are separated and the bases are liberated from the salts.

In view of the close relationship beween the free compounds and the compounds in the form of their salts, whenever a compound is referred to in this context, a corresponding salt is also intended, provided such is possible or appropriate under the circumstances.

The compounds, including their salts, can also be obtained in the form of their hydrates, or include other solvents used for their crystallization.

The pharmaceutical compositions according to the invention are those suitable for enteral, such as oral or rectal, transdermal and parenteral administration to mammals, including man, for blockade of the N-methyl-D-aspartate sensitive excitatory amino acid receptor and for the treatment of diseases responsive to blockade of the N-methyl-D-aspartate sensitive excitatory amino acid receptor, such as convulsive disorders and anxiety, comprising an effective amount of a pharmacologically active compound of the invention, alone or in combination with one or more pharmaceutically acceptable carriers.

The pharmacologically active compounds of the invention are useful in the manufacture of pharmaceutical compositions comprising an effective amount thereof in conjunction or admixture with excipients or carriers suitable for either enteral or parenteral application. Preferred are tablets and gelatin capsules comprising the active ingredient together with a) diluents, e.g. lactose, dextrose, sucrose, mannitol, sorbitol, cellulose and/or glycine; b) lubricants, e.g. silica, talcum, stearic acid, its magnesium or calcium salt and/or polyethyleneglycol ; for tablets also c) binders e.g. magnesium aluminum silicate, starch paste, gelatin, tragacanth, methylcellulose, sodium carboxymethylcellulose and/or polyvinylpyrrolidone ; if desired d) disintegrants, e.g. starches, agar, alginic acid or its sodium salt, or effervescent mixtures ; and/or e) absorbents, colorants, flavors and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1 to 75%, preferably about 1 to 50%, of the active ingredient.

Suitable formulations for transdermal application include an effective amount of a compound of formula I with carrier. Advantageous carriers include absorbable pharmacologically acceptable solvents to assist passage throught the skin of the host. Characteristically, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device of the skin.

The invention also relates to a method of blocking the N-methyl-D-aspartate sensitive excitatory amino acid receptor in mammals, and to a method of treatment of disorders in mammals, e.g. such responsive to blockade of the N-methyl-D-aspartate excitatory amino acid receptor, such as convulsive disorders and anxiety, using an effective amount of a compound of the invention as a pharmacologically active substance, preferably in the form of above-cited pharmaceutical compositions. The dosage of active compound administered is dependent on the species of warm-blooded animal (mammal), the body weight, age and individual condition, and on the form of administration.

A unit dosage for a mammal of about 50 to 70 kg may contain between about 5 to 100 mg of the active ingredient.

The following examples are intended to illustrate the invention and are not to be construed as being limitations thereon. Temperatures are given in degrees Centigrade. If not mentioned otherwise, all evaporations are performed under reduced pressure, preferably between about 2 and 13 kPa.

Example 1 :

A mixture of 1.19 g of 4-(diethylphosphonomethyl)-2-pyridine-carboxamide and 0.75 g of platinum oxide in 30 ml of acetic acid is hydrogenated at 300 kPa for 6 hours at room temperature. The reaction mixture is filtered and the solvent is removed in vacuo. The residue is dissolved in methylene chloride and washed with a small volume of saturated sodium bicarbonate solution. After drying over magnesium sulfate, a methylene chloride solution of a 2 : 1 mixture of cis and trans 4-(diethylphosphonomethyl)-2-piperidinecarboxamide is obtained. Within 3 days at room temperature this mixture isomerizes to the more stable cis isomer. Removal of the solvent in vacuo affords cis-4-(diethylphosphono-methyl)-2-piperidinecarboxamide as an oil, which is converted to the hydrochloride salt, melting at 155-158°.

The starting material is prepared as follows : A mixture of 2.2 g of 4-(diethylphosphonomethyl)pyridine [Roczniki Chem. 38, 625 (1964) ; Chem. Abst. 61, 10703], 2.2 g of m-chloroperbenzoic acid and 30 ml of chloroform is stirred at room temperature for 16 hours. The solvent is removed in vacuo and water is added. After washing the aqueous layer with ether, the water is removed in vacuo to afford 4-(diethylphosphonomethyl)-pyridine-N-oxide.

A mixture of 1.2 g of 4-(diethylphosphonomethyl)pyridine-N-oxide, 1.35 ml of triethylamine and 2.6 ml of trimethylsilylcyanide is heated at 90° for 1 hour. The volatiles are removed in vacuo and the residue is dissolved in ethyl acetate and washed with a small volume of aqueous sodium bicarbonate. After drying over magnesium sulfate the solvent is removed in vacuo to afford 2-cyano-4-(diethylphosphonomethyl)pyridine as an oil.

A mixture of 1.10 g of 2-cyano-4-(diethylphosphonomethyl)pyridine and 4.5 ml of concentrated sulfuric acid is heated at 90° for 5 minutes. The reaction mixture is poured onto ice and neutralized with 10% sodium hydroxide. The resulting precipitate is collected to afford 4-(diethyl-phosphonomethyl)-2-pyridinecarboxamide melting at 140-142°.

Example 2 :

A solution of 3.0 g of cis-4-(diethylphosphonomethyl)-2-piperidinecarboxamide in 90 ml of 20% hydrochloric acid is heated under reflux with stirring for 16 hours. After removal of the solvent, the residue is crystallized from ethyl acetate/ethanol to afford cis-4-phosphonomethyl-2-piperidinecarboxylic acid hydrochloride, melting at 284-285° (dec).

Example 3 :

A mixture of 330 mg of 4-phosphonomethyl-2-pyridinecarboxylic acid, 150 mg of platinum oxide, 20 ml of acetic acid and 100 ml of water is hydrogenated at 300 kPa at room temperature for 20 hours. The solvent is removed in vacuo to afford a 2 : 1 mixture of cis and trans 4-phosphono-methyl-2-piperidinecarboxylic acid. The residue is dissolved in aqueous hydrochloric acid and the solvent removed in vacuo to afford a 2 : 1 mixture of cis and trans 4-phosphonomethyl-2-piperidinecarboxylic acid hydrochloride melting at 145° (dec).

The starting material is prepared as follows : A mixture of 350 mg 2-cyano-4-diethylphosphonomethylpyridine and 10 ml of 20% hydrochloric acid is refluxed for 16 hours. After removal of the solvent in vacuo the residue is triturated with 95% ethanol to afford 4-phosphonomethyl-2-pyridinecarboxylic acid melting at 265-268° (dec).

Example 4 :

Prepared essentially according to the procedures described in the previous examples are :

a) cis and trans 5-phosphonomethyl-2-piperidinecarboxylic acid hydrochloride, m.p. above 250° ;
b) cis and trans 3-phosphonomethyl-2-piperidinecarboxylic acid hydrochloride, m.p. above 250° ;
c) cis-3-(2-phosphonoethyl)-2-piperidinecarboxylic acid, m.p. 160-190° (dec) ;
d) cis-5-(2-phosphonoethyl)-2-piperidinecarboxylic acid hydrochloride, m.p. 125° (dec) ;
e) cis-4-(2-phosphonoethyl)-2-piperidinecarboxylic acid ;
f) cis-4-(3-phosphonopropyl)-2-piperidinecarboxylic acid, m.p. 287° (dec) ;
g) cis-4-(1-methyl-1-phosphonoethyl)-2-piperidinecarboxylic acid hydrochloride, m.p. 250° (dec) ;
h) cis-4-(1-phosphonoethyl)-2-piperidinecarboxylic acid, m.p. 220° (dec), e.g. via 4-(1-phosphonoethyl)-2-pyridinecarboxylic acid, m.p. 264-265° (dec) ;
i) 5-(3-phosphonopropyl)-2-piperidinecarboxylic acid, e.g. via 5-(3-phosphonopropyl)-2-pyridinecarboxylic acid hydrochloride, m.p. 210-220° (dec) ;
j) cis-3-(3-phosphonopropyl)-2-piperidinecarboxylic acid, via 3-(3-phosphonopropyl)-2-pyridinecarboxylic acid, m.p. 230-235° (dec) ;

k) 3-methyl-4-phosphonomethyl-2-piperidinecarboxylic acid, m.p. 325° (dec), starting from 3-methyl-4-chloromethylpyridine via reduction of 3-methyl-4-phosphonomethylpyridinecarboxylic acid ;

l) cis-6-methyl-4-phosphonomethyl-2-piperidinecarboxylic acid, m.p. 260-265°, starting from 2-methyl-4-chloromethylpyridine ;

m) cis-5-methyl-4-phosphonomethyl-2-piperidinecarboxylic acid, m.p. above 250°, starting from 3-methyl-4-chloromethylpyridine via reduction of 5-methyl-4-phosphonomethyl-2-pyridinecarboxylic acid, m.p. 295° (dec).

Certain starting materials are prepared as follows :

1. Treatment of 3-(diethylphosphonomethyl)pyridine (Biochemistry 19, 3400) with m-chloroperbenzoic acid followed by trimethylsilyl cyanide gives both 3-(diethylphosphonomethyl)-2-cyanopyridine and 5-(diethylphosphonomethyl)-2-cyanopyridine, the starting materials for compounds under a) and b).

2. Similarly, 3-(2-diethylphosphonoethyl)pyridine (Biochemistry 19, 3400) is converted to the corresponding starting materials for compounds under c) and d) ; 4-(2-diethylphosphonoethyl)pyridine (Chem. Abst. 89, 35665) is converted to 4-(2-diethylphosphonoethyl)-2-cyanopyridine, the starting material for compound under e).

3. Reaction of 3-(4-pyridinyl)propyl chloride with diethyl phosphite (diethyl phosphonate) and sodium hydride in toluene yields 4-(3-di-ethylphosphonopropyl)pyridine which is converted to 4-(3-diethyl-phosphonopropyl)-2-cyanopyridine according to the procedure described hereinabove, the starting material for compound under f).

4. A mixture of 1.0 g of 4-(diethylphosphonomethyl)pyridine and 0.5 g of 50% sodium hydride in 4 ml of dimethylformamide and 16 ml of tetrahydrofuran is heated under reflux for 30 minutes. Methyl iodide (0.52 ml) is added and heating under reflux is continued for 30 minutes. The reaction mixture is evaporated to dryness and the residue is chromatographed on silica gel with methanol/methylene chloride (1 : 10) as the eluent to give 4-(1-methyl-1-diethylphosphonoethyl)pyridine which is converted to 2-cyano-4-(1-methyl-1-diethylphosphonoethyl)pyridine, the starting material for compound under g).

5. Similarly treatment of 4-(diethylphosphonomethyl)pyridine as above with 1 mole equivalent of methyl iodide yields 4-(1-diethylphosphono-ethyl)pyridine, the starting material for compound under h).

Example 5 :

a) A solution of 370 mg of cis-4-phosphonomethyl-2-piperidinecarboxylic acid in 15 ml of saturated ethanolic hydrochloric acid is heated under reflux for 16 hours. The solvent is removed in vacuo. A solution of the residue in ethanol is treated with 0.21 ml of propylene oxide and evaporated to dryness to yield cis-4-phosphonomethyl-2-piperidine-carboxylic acid ethyl ester, m.p. 230-235° (dec).

Similarly prepared are :

b) cis-4-phosphonomethyl-2-piperidinecarboxylic acid methyl ester, m.p. 198-200° ;

c) cis-4-phosphonomethyl-2-piperidinecarboxylic acid n-butyl ester, m.p. 241-244° ;

d) cis-4-(3-phosphonopropyl)-2-piperidinecarboxylic acid ethyl ester, m.p. 197-202° ;

e) cis-4-phosphonomethyl-2-piperidinecarboxylic acid n-propyl ester, m.p. 245-249° ;

f) cis-4-phosphonomethyl-2-piperidinecarboxylic acid isobutyl ester, m.p. 254-259°.

Example 6 :

A solution of 278 mg of cis-4-diethylphosphonomethyl-2-piperidinecarboxamide in 5 ml of methylene chloride to which is added 0.43 ml of trimethylsilyl iodide is stirred at room temperature for 16 hours. The mixture is evaporated to dryness, the residue is dissolved in water, and the solution is evaporated to dryness. A solution of the resulting solid in ethanol is treated with 0.21 ml of propylene oxide to yield cis-4-phosphonomethyl-2-piperidinecarboxamide, m.p. 295-298° (dec).

Example 7 :

a) To a solution of 200 mg of cis-4-phosphonomethyl-2-piperidine-carboxylic acid, 96 mg of sodium carbonate in 5 ml of water and 2.31 ml of 1N aqueous sodium hydroxide is added 119 mg of benzoyl chloride. The reaction mixture is stirred at room temperature for 16 hours and the solvent is removed in vacuo. The residue is dissolved in ethanol, the solution is filtered and evaporated to dryness. Crystallization from ethanol/ethyl acetate affords cis-1-benzoyl-4-phosphonomethyl-2-piperidinecarboxylic acid, m.p. 135-145° (dec).

b) Similarly prepared is cis-1-benzyloxycarbonyl-4-phosphonomethyl-2-piperidinecarboxylic acid methyl

ester.

c) Similarly prepared is cis-1-(3-phenylpropionyl)-4-phosphonomethyl-2-piperidinecarboxamide, m.p. 95-100°, via cis-4-phosphonomethyl-2-piperidinecarboxamide.

Example 8 :

A mixture of 357 mg of cis-1-benzyloxycarbonyl-4-phosphono-methyl-2-piperidinecarboxylic acid, 550 mg of diisopropylethylamine and 600 mg of chloromethyl pivalate in 2 ml of dimethylformamide is heated at 80° for 2 hours. The reaction mixture is evaporated to dryness at 70°/13 Pa. The residue is dissolved in ethyl acetate, the solution is washed with water and evaporated to dryness to yield cis-1-benzyloxy-carbonyl-4-[di(pivaloyloxymethyl)phosphonomethyl]-2-piperidinecarboxylic acid pivaloyloxymethyl ester (after chromatography on silica gel using ether/methylene dichloride as the eluent). The product is dissolved in 40 ml of ethanol and hydrogenated in the presence of 10% palladium on charcoal to yield cis-4-[di(pivaloyloxymethyl)phosphonomethyl]-2-piperidinecarboxylic acid pivaloyloxymethyl ester.

The starting material is prepared by treatment of cis-4-phosphono-methyl-2-piperidinecarboxylic acid with benzyl chloroformate under standard amino acid acylation conditions, e.g. as described in example 7.

Example 9 :

a) A mixture of 500 mg of 4-(diethylphosphonomethyl)quinoline-2-carbox-amide and 1 g of 10% palladium on charcoal in 75 ml of methanol is hydrogenated at 300 kPa for 24 hours, filtered and evaporated to dryness. Chromatography on silica gel using methanol/methylene chloride (1 : 10) as eluent yields cis-4-(diethylphosphonomethyl)-1,2,3,4-tetrahydroquinoline-2-carboxamide.

The starting material is prepared as follows : To a stirred solution of 10 g of 4-quinolinecarboxaldehyde in 400 ml of absolute ethanol at room temperature is added 2.4 g of sodium borohydride. After 45 minutes, 20 ml of water is added and the reaction mixture is stirred for an additional 20 minutes. Acetic acid (20 ml) is added slowly. The reaction mixture is evaporated to a small volume and partitioned between water and methylene chloride. The organic layer is washed with saturated potassium carbonate, saturated sodium chloride solution, dried over sodium sulfate and evaporated to dryness. The residue is purified by chromatography on silica gel using methanol/methylene chloride (1 : 10) as eluent to yield 4-hydroxymethylquinoline ; $R_f$ = 0.59.

A solution of 4.84 ml of methanesulfonyl chloride in 20 ml of methylene chloride is slowly added over 20 minutes to a solution of 8.29 g of 4-hydroxymethylquinoline and 11 ml of triethylamine in 200 ml of methylene chloride at 0°. After completion of the reaction at room temperature, the reaction mixture is partitioned between methylene chloride and saturated potassium carbonate solution. The organic layer is dried over sodium sulfate and evaporated to dryness to yield 4-(methanesulfonyloxymethyl)quinoline as an oil.

To a solution of 1.62 g of sodium hydride (from 3.24 g of a 50% sodium hydride dispersion in mineral oil) in 100 ml of toluene is added dropwise a solution of 8 ml of diethyl phosphite in 20 ml of toluene. The reaction mixture is heated to 80°, a solution of the above obtained 4-(methanesulfonyloxymethyl)quinoline in 90 ml of toluene and 10 ml of methylene chloride is added. After 20 minutes a second portion of 1.2 g of sodium hydride is added and the reaction mixture is stirred for an additional 10 minutes. The reaction mixture is then evaporated to dryness, the product is partitioned between water and ethyl acetate. The ethyl acetate extract is washed with saturated sodium chloride solution, dried over sodium sulfate and evaporated to dryness. The residue is chromatographed on silica gel using methanol/methylene chloride (5 : 95) as eluent to yield 4-(diethylphosphonomethyl)quinoline ; $R_f$ = 0.3.

To a solution of 5.7 g of 4-(diethylphosphonomethyl)quinoline in 100 ml of methylene chloride at room temperature is added 3.2 g of m-chloro-perbenzoic acid. After 45 minutes an additional 1.5 g portion of m-chloroperbenzoic acid is added, and the reaction mixture is stirred for one hour. The reaction mixture is washed with saturated potassium carbonate solution, dried over sodium sulfate and evaporated to dryness to give 4-(diethylphosphonomethyl)quinoline-N-oxide.

A mixture of 5.9 g of 4-(diethylphosphonomethyl)quinoline-N-oxide, 15 ml of trimethylsilyl cyanide and 7 ml of triethylamine is heated at 80° for 1 hour and evaporated to dryness. The residue is purified by chromatography on silica gel using methanol/methylene chloride (5 : 95) as eluent to yield 4-(diethylphosphonomethyl)-2-cyanoquinoline as an oil.

A solution of 5.9 g of 4-(diethylphosphonomethyl)-2-cyanoquinoline in 24 ml of concentrated sulfuric acid is heated at 80° for 5 minutes, cooled to 0° and added slowly to an ice-cooled mixture of 50 g of sodium carbonate in 50 ml of water and 100 ml of methylene chloride. The organic layer is separated, dried over sodium sulfate and evaporated to dryness to yield 4-(diethylphosphonomethyl)quinoline-2-carboxamide.

b) Similarly prepared is cis-4-(3-diethylphosphonopropyl)-1,2,3,4-tetra-hydroquinoline-2-carboxamide.

Example 10 :

a) A solution of 220 mg of cis-4-(diethylphosphonomethyl)-1,2,3,4-tetra-hydroquinoline-2-carboxamide in 50 ml of 6N aqueous hydrochloric acid is heated under reflux for 14 hours, and evaporated to dryness. The residue is dried under high vacuum at 100° for 48 hours to yield cis-4-phosphono-methyl-1,2,3,4-tetrahydroquinoline-2-carboxylic acid, m.p. 171-173°.

b) Similarly prepared is cis-4-(3-phosphonopropyl)-1,2,3,4-tetra-hydroquinoline-2-carboxylic acid hydrochloride.

Example 11 :

a) Treatment of cis-4-phosphonomethyl-1,2,3,4-tetrahydroquinoline-2-carboxylic acid first with benzyl chloroformate and then with chloromethyl pivalate under conditions essentially as described in example 8 including hydrogenolysis yields 4-[di-(pivaloyloxymethyl)phosphono-methyl]-1,2,3,4-tetrahydroquinoline-2-carboxylic acid pivaloyloxymethyl ester.

b) Treatment of cis-4-phosphonomethyl-1,2,3,4-tetrahydroquinoline-2-carboxylic acid with ethanolic hydrochloric acid under conditions essentially as described in example 5 yields cis-4-phosphonomethyl-1,2,3,4-tetrahydroquinoline-2-carboxylic acid ethyl ester.

Example 12 :

A mixture of 400 mg of 4-phosphonomethylquinoline-2-carboxylic acid hydrochloride, 150 mg of platinum oxide, 20 ml of acetic acid and 100 ml of water is hydrogenated at 300 kPa at room temperature for 20 hours to afford cis-4-phosphonomethylperhydroquinoline-2-carboxylic acid ; the hydrochloride salt melts at 230-250° (dec).

The starting material is prepared as follows : A solution of 4-(d-iethylphosphonomethyl)quinoline-2-carboxamide in 6N hydrochloric acid is heated under reflux overnight to yield 4-phosphonomethylquinoline-2-carboxylic acid hydrochloride, m.p. 218-221°.

Example 13 :

A mixture of 2.0 g of 4-(diethylphosphonomethylidene)-N-t-butoxycarbonylpyrrolidine-2-carboxylic acid methyl and ethyl ester, and 800 mg of 10% palladium on charcoal catalyst in 30 ml of ethanol is hydrogenated at 300 kPa and room temperature for 18 hours. The reaction mixture is filtered free of catalyst, the catalyst is washed with methylene chloride, the combined filtrate is evaporated to dryness to yield a mixture of N-t-butoxycarbonyl-4-diethylphosphonomethyl-pyrrolidine-2-carboxylic acid methyl and ethyl ester.

The above product is treated with 10 ml trifluoroacetic acid for 5 minutes, the trifluoroacetic acid is removed under vacuum. A solution of the residue in 30 ml of 6N hydrochloric acid is heated under reflux for 6 hours and evaporated to dryness. A solution of the residue in 15 ml ethanol is treated with 1 ml of propylene oxide. The resulting precipitate is filtered off to yield 4-phosphonomethylpyrrolidine-2-carboxylic acid (4-phosphonomethylproline) ; $^1$H-NMR ($D_2O$) : 4.53, 3.82, 2.88, 2.80, 2.0 ppm.

The starting material is prepared as follows : A mixture of 10.0 g of 4-hydroxyproline, 13 ml of 6N sodium hydroxide solution and 19.0 g of di-t-butyl carbonate is stirred vigorously at room temperature for 24 hours. Concentrated hydrochloric acid (6.5 ml) is added and the reaction mixture is extracted with 30 ml of chloroform. The chloroform is discarded, the oil is separated from the aqueous layer to give N-t-butoxycarbonyl-4-hydroxyproline. A solution thereof in 70 ml of methanol is treated with ethereal diazomethane (prepared from 20 g of N-methyl-N-nitrosourea, 60 ml of 40% aqueous potassium hydroxide and 200 ml of ether) to give N-t-butoxycarbonyl-4-hydroxyproline methyl ester.

A mixture of 12.7 g of N-t-butoxycarbonyl-4-hydroxyproline methyl ester and 44.7 g of pyridinium chlorochromate in 150 ml of methylene chloride is stirred at room temperature for 30 hours, diluted with 200 ml of ether and filtered through Florisil washing with ether (500 ml). The solution is evaporated to dryness, the residue is purified by flash chromatography on silica gel using ethyl acetate/hexane (4 :6) as eluent to give N-t-butoxycarbonyl-4-oxoproline methyl ester.

A solution of 2.28 M n-butyl lithium (4.6 ml) is added at −78° to a solution of 3.07 g of bis(diethylphosphono)methane in 50 ml of dry tetrahydrofuran. After 5 minutes, a solution of 2.5 g of N-t-butoxy-carbonyl-4-oxoproline methyl ester is added. The reaction mixture is heated under reflux for 20 hours. The volume is

reduced to 20 ml and the product is partitioned between methylene chloride and 2N hydrochloric acid. The methylene chloride extract is dried and evaporated to dryness and the residue is purified by flash chromatography on silica gel eluting with methylene chloride/methanol (95 : 5) to obtain a mixture of 4-(diethylphosphonomethylidene)-N-t-butoxycarbonylpyrrolidine-2-carboxylic acid methyl and ethyl ester.

Example 14 :

Preparation of an injectable formulation containing 10 mg of the active ingredient per 5 ml of solution having a formula as follows :

| | |
|---|---|
| cis-4-phosphonomethyl-2-piperidine-carboxylic acid hydrochloride | 10.0 g |
| Propylparaben | 0.5 g |
| Water for injection q.s. | 5000.0 ml |

The active ingredient and preservative are dissolved in 3500 ml of water for injection and the solution is diluted to 5000 ml. The solution is filtered through a sterile filter and filled into injection vials under sterile conditions each vile containing 5 ml of the solution.

Example 15 :

A solution of 2.0 g of cis-1-benzyloxycarbonyl-4-phosphonomethyl-2-piperidinecarboxylic acid in 10 ml of methylene chloride is treated with ethereal diazomethane until a persistent yellow color is obtained. After removal of solvent the triester is heated at 50° in 10 ml of tetrahydrofuran and 10 ml of 1N sodium hydroxide. After acidification the product is extracted with ether, the organic layer dried and evaporated to yield cis-1-benzyloxycarbonyl-4-dimethylphosphonomethyl-2-piperidinecarboxylic acid.

Example 16 :

A solution of 1.0 g of cis-1-benzyloxycarbonyl-4-dimethyl-phosphonomethyl-2-piperidinecarboxylic acid in 30 ml of ethanol is hydrogenated at 300 kPa over 500 mg of 10% palladium on carbon catalyst in 30 ml of ethanol. Filtration and removal of solvent affords cis-4-di-methylphosphonomethyl-2-piperidine-carboxylic acid as an amorphous solid.

Example 17 :

A solution of 3.38 g of cis-1-benzyloxycarbonyl-4-dimethyl-phosphonomethyl-2-piperidinecarboxylic acid in 35 ml of methylene chloride is treated with 1.42 g of 1,1'-carbonyldiimidazole and 1.3 g of (+)-α-methylbenzylamine. After stirring for 16 hours, the reaction mixture is washed with 1N hydrochloric acid and saturated sodium bi-carbonate solution, dried, and the solvent removed. Separation of the resulting diastereomers by high pressure liquid chromatography (HPLC) using a reverse phase $C_{18}$ column and acetonitrile/water (2 : 3) as eluent affords the two diastereomers of cis-1-benzyloxycarbonyl-4-dimethyl-phosphonomethyl-2-piperidine-(N-1-phenylethyl)carboxamide.

Hydrogenation of the first eluted diastereomer in ethanol with palladium on charcoal catalyst for 4 hours at 300 kPa affords the corresponding cis-4-dimethylphosphonomethyl-2-piperidine-(N-1-phenylethyl)carboxamide. Treatment with 6N hydrochloric acid under reflux for 24 hours affords (–)-cis-4-phosphonomethyl-2-piperidinecarboxylic acid, $[\alpha]^{25}_{D} = -5.93°$ ($H_2O$), the pharmacologically more active enantiomer of the compound of example 2.

The corresponding (+)-cis-4-phosphonomethyl-2-piperidinecarboxylic acid, the less active enantiomer, is similarly obtained.

Example 18 :

A mixture of 600 mg of cis-1-benzyloxycarbonyl-4-dimethyl-phosphonomethyl-2-piperidinecarboxylic acid

methyl ester in 10 ml of methylene chloride is treated with 538 mg of trimethylsilyl iodide at room temperature to yield cis-1-benzyloxycarbonyl-4-phosphonomethyl-2-piperidinecarboxylic acid methyl ester as an oil.

Example 19 :

A mixture of 500 mg of cis-4-diethylphosphonomethyl-2-piperidinecarboxamide, 3 ml of 37% aqueous formaldehyde, 50 ml of methanol and 500 mg of 10% palladium on carbon catalyst is hydrogenated at 300 kPa for 16 hours. The solvent is removed and the residue chromatographed on silica gel with 10% methanol/methylene chloride as the eluent to afford cis-1-methyl-4-diethylphosphonomethyl-2-piperidinecarboxamide. This product is refluxed for 16 hours in 6N hydrochloric acid followed by treatment with propylene oxide to yield cis-1-methyl-4-phosphonomethyl-2-piperidinecarboxylic acid, m.p. 256-260°.

Example 20 :

A solution of 1.0 g of cis-4-diethylphosphonomethyl-2-piperidinecarboxylic acid ethyl ester is reacted with 552 mg of N-t-butoxycarbonylglycine in the presence of N, N'-dicyclohexyl-carbodiimide in 10 ml of methylene chloride of room temperature for 16 hours to yield cis-1-[α-(t-butoxycarbonylamino)acetyl]-4-diethylphosphonomethyl-2-piperidinecarboxylic acid ethyl ester as an oil. Treatment with trimethylsilyl iodide yields cis-1-(α-aminoacetyl)-4-phosphonomethyl-2-piperidinecarboxylic acid ethyl ester, m.p. 150-155° (dec).

Example 21 :

a) N-t-butoxycarbonyl-4-(2-diethylphosphonoethyl)pyrrolidine-2-carboxylic acid methyl ester (0.45 g) is hydrolyzed as described in example 13 to yield 4-(2-phosphonoethyl)pyrrolidine-2-carboxylic acid ; $^1H$-NMR ($D_2O$) : 4.22, 3.45, 2.95, 2.54, 2.4 ppm (1H each).

The starting material is prepared as follows: To a suspension of 3.44 g of benzyloxycarbonylmethyltriphenylphosphonium bromide in 20 ml of tetrahydrofuran is added 10 ml 0.65 M potassium hexamethyldisilazide solution in toluene followed by 1.5 g N-t-butoxycarbonyl-4-oxoproline methyl ester in 4 ml of tetrahydrofuran. After refluxing for 72 hours the solvent is evaporated and the residue flash chromatographed with ethyl acetate/hexane (2 : 8) to afford a mixture of cis and trans N-t-butoxy-carbonyl-4-(2-oxo-2-benzyloxyethylidene)pyrrolidine-2-carboxylic acid methyl ester.

A solution of 0.58 g of the above product in 15 ml of methanol is hydrogenated at 300 kPa for 3 hours in the presence of 400 mg of 10% Pd/C. The reaction mixture is filtered through Celite, the solvent evaporated and the residue flash chromatographed with methanol/methylene chloride (1 : 9) to afford N-t-butoxycarbonyl-4-(carboxymethyl)pyrrolidine-2-carboxylic acid methyl ester.

To 0.67 g N-t-butoxycarbonyl-4-(carboxymethyl)pyrrolidine-2-carboxylic acid methyl ester in 1 ml THF is added at 0° 3.5 ml of borane (1M) in THF. After stirring 0.5 hour, 1 ml of water is added, the solvent is evaporated and the residue is flash chromatographed with ethyl acetate/hexane (75 : 25) to afford N-t-butoxycarbonyl-4-(2-hydroxyethyl)-pyrrolidine-2-carboxylic acid methyl ester.

To a solution of 0.58 g N-t-butoxycarbonyl-4-(2-hydroxyethyl)pyrrolidine-2-carboxylic acid methyl ester in 6 ml methylene chloride is added at 0° 0.577 g triphenylphosphine followed by 0.388 g N-bromosuccinimide. After stirring 0.5 hour, the solvent is evaporated and the residue flash chromatographed using ethyl acetate/hexane (35 : 65) to afford N-t-butoxy-carbonyl-4-(2-bromoethyl)pyrrolidine-2-carboxylic acid methyl ester.

A mixture of 0.60 g N-t-butoxycarbonyl-4-(2-bromoethyl)pyrrolidine-2-carboxylic acid methyl ester and 3 ml triethyl phosphite is refluxed for 2 hours. The excess triethyl phosphite is distilled off under vacuum and the residue is flash chromatographed with methylene chloride/methanol (95 : 5) to afford N-t-butoxycarbonyl-4-(2-diethylphosphonoethyl)-pyrrolidine-2-carboxylic acid methyl ester.

b) Similarly obtained is (+)-cis-4-(2-phosphonoethyl)pyrrolidine-2-carboxylic acid, $[\alpha]_D^{25}$ = +23.7°, starting with the D- proline derivative.

c) Similarly prepared is 3-(2-phosphonoethyl)pyrrolidine-2-carboxylic acid.

Example 22 :

a) A stirred solution of 5.3 ml of bis(diethylphosphono)methane in 30 ml of anhydrous tetrahydrofuran (THF) under nitrogen is cooled to −78° and 8.5 ml of 2.5 M butyl lithium is added dropwise. After stirring 5 minutes a solution of 4.64 g of 1-ethoxycarbonyl-3-oxopyrrolidine-2-carboxylic acid ethyl ester [J. Am. Chem. Soc. 86,

5297 (1964)] in 30 ml dry THF is added dropwise rapidly. The solution is heated at reflux for 18 hours, cooled to room temperature and concentrated ; 40 ml of 1N hydrochloric acid is added and the mixture is extracted with 100 ml of methylene chloride. The organic fraction is washed with 25 ml water, dried over sodium sulfate, filtered and concentrated. Purification by flash chromatography using ethyl acetate as solvent yields 1-ethoxycarbonyl-3-(diethylphosphonomethyl)-2,5-dihydropyrrole-2-carboxylic acid ethyl ester.

Example 23 :

A solution of 2.75 g of 1-ethoxycarbonyl-3-(diethylphos-phonomethyl)-2,5-dihydropyrrole-2-carboxylic acid ethyl ester in 35 ml ethanol and 1.5 g 10% Pd/C is hydrogenated at 300 kPa to yield cis and trans 1-ethoxycarbonyl-3-(diethylphosphonomethyl)pyrrolidine-2-carboxylic acid ethyl ester.

Example 24 :

a) A solution of 2.7 g of cis and trans 1-ethoxycarbonyl-3-(diethyl-phosphonomethyl)pyrrolidine-2-carboxylic acid ethyl ester is heated under reflux with 6N hydrochloric acid for 6 hours to yield 3-(phosphonomethyl)pyrrolidine-2-carboxylic acid hydrochloride ; $^1$H-NMR ($D_2O$) : 4.78, 4.40, 3.95, 3.83, 3.72, 3.20, 3.01, 2.78, 2.69 ppm.

b) Similarly hydrolysis of 1-ethoxycarbonyl-3-(diethylphosphonomethyl)-2,5-dihydropyrrole-2-carboxylic acid ethyl ester affords 3-(phosphono-methyl)-2,5-dihydropyrrole-2-carboxylic acid hydrochloride which on treatment with propylene oxide yields the free amino acid, m.p. 215° (dec)

## Claims

**Claims the Contracting States : BE, CH, DE, FR, GB, IT, LI, LU, NL, SE**

1. A compound of the formula I,

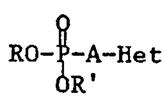

(I),

wherein R and R′ independently represent hydrogen, $C_1$-$C_4$alkyl, benzyl, benzyl substituted on phenyl by halogen, $C_1$-$C_4$alkyl or $C_1$-$C_4$alkoxy, $C_2$-$C_7$alkanoyloxymethyl or $C_2$-$C_7$alkanoyloxymethyl substituted on oxymethyl by $C_1$-$C_4$alkyl or $C_3$-$C_8$cycloalkyl, A denotes $C_1$-$C_4$alkylene and Het represents a 2-$R^1$-pyrrolidinyl, 2-$R^1$-2,5-dihydropyrrolyl, 2-$R^1$-1,2,3,6-tetrahydropyridinyl, 2-$R^1$-1,2,5,6-tetrahydropyridinyl, 2-$R^1$-piperidinyl, 2-$R^1$-tetrahydroquinolinyl, 2-$R^1$-perhydroquinolinyl, 2-$R^1$-carboxy-2,3-di-hydroindolyl or 2-$R^1$-perhydroindolyl group which may be additionally N-substituted by $C_1$-$C_4$alkyl, phenyl-$C_1$-$C_4$alkyl, $C_2$-$C_7$alkanoyl, benzoyl, $C_1$-$C_4$alkoxycarbonyl, benzyloxycarbonyl, phenylacetyl, 3-phenylpropionyl or α-amino-$C_2$-$C_4$alkyl, C-substituted in the heterocyclic 5- or 6-ring by $C_1$-$C_4$alkyl or phenyl-$C_1$-$C_4$alkyl and/or, if present, in the benzo or cyclohexano ring by $C_1$-$C_4$alkyl, $C_1$-$C_4$alkoxy, halo or trifluoromethyl or a salt thereof.

2. A compound according to claim 1 of the formula II

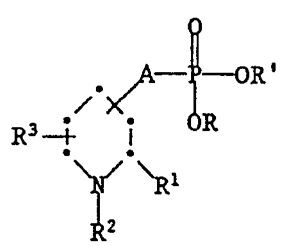

(II)

or a compound of formula II with a double bond present between C-3 and C-4 or between C-4 and C-5 of the piperidinyl ring, in which the phosphono bearing chain is attached at the 3-, 4-, or 5-position of the piperidinyl or tetrahydropyridinyl ring, and wherein R and R′ independently represent hydrogen, $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, cyclohexyl or cyc-

lopentyl, $R^1$ represents carboxy, carbamoyl, N-mono- or N,N-di-$C_1$-$C_4$alkylcarbamoyl or $C_1$-$C_4$alkoxycarbonyl, $R^2$ and $R^3$ represent hydrogen or $C_1$-$C_4$alkyl and A represents $C_1$-$C_4$alkylene ; or a pharmaceutically acceptable salt thereof.

3. A compound of formula II according to claim 2 wherein R and R′ independently represent hydrogen, $C_2$-$C_4$alkanoyloxymethyl or $C_2$-$C_4$alkanoyloxy-methyl substituted on oxymethyl by $C_1$-$C_4$alkyl, $R^1$ represents carboxy, carbamoyl or $C_1$-$C_4$alkoxycarbonyl, $R^2$ and $R^3$ represent hydrogen and A is at the 4-position and represents $C_1$-$C_4$alkylene ; or a pharmaceutically acceptable salt thereof.

4. A compound according to claim 2 of the formula III

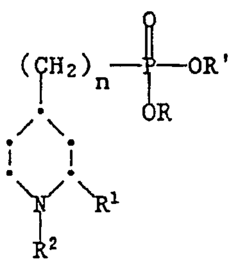

(III)

wherein n represents the integer 1, 2 or 3 ; R and R′ independently represent hydrogen, $C_1$-$C_4$alkyl, benzyl, $C_2$-$C_4$alkanoyloxymethyl or $C_2$-$C_4$-alkanoyloxymethyl substituted on oxymethyl by $C_1$-$C_4$alkyl, $R^1$ represents carboxy, $C_1$-$C_4$alkoxycarbonyl or carbamoyl and $R^2$ represents hydrogen, $C_1$-$C_4$alkyl, $C_2$-$C_4$alkanoyl or benzoyl; or a pharmaceutically acceptable salt of said compound having a salt-forming functional grouping.

5. A compound of formula III according to claim 4 wherein n represents the integer 1, 2 or 3 ; R and R′ both represent hydrogen or $C_2$-$C_4$alkanoyloxymethyl ; or one of R and R′ represents hydrogen and the other of R and R′ represents $C_1$-$C_4$alkyl benzyl, $C_2$-$C_4$alkanoyloxymethyl or $C_2$-$C_4$-alkanoyloxymethyl substituted on oxymethyl by $C_1$-$C_4$alkyl, $R^1$ represents carboxy or $C_1$-$C_4$alkoxycarbonyl and $R^2$ represents hydrogen ; or a pharmaceutically acceptable salt thereof.

6. A compound of formula III according to claim 4 wherein n represents the integer 1, 2 or 3 ; R and R′ represent hydrogen, $R^1$ represents carboxyor $C_1$-$C_4$alkoxycarbonyl and $R^2$ represents hydrogen ; or a pharmaceutically acceptable salt thereof.

7. A compound according to claim 6 being cis-4-phosphonomethyl-2-pipe-ridinecarboxylic acid or a pharmaceutically acceptable salt thereof.

8. A compound according to claim 6 being cis-4-(3-phosphonopropyl)-2-piperidinecarboxylic acid or a pharmaceutically acceptable salt thereof.

9. A compound according to claim 6 being cis-4-phosphonomethyl-2-pipe-ridinecarboxylic acid ethyl ester or a pharmaceutically acceptable salt thereof.

10. A compound according to claim 1 of the formula IV

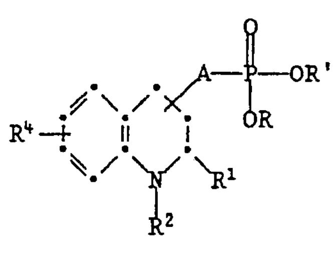

(IV)

or a perhydro derivative thereof, wherein R and R′ independently represent hydrogen, $C_1$-$C_4$alkyl, benzyl, benzyl substituted on phenyl by halogen, $C_1$-$C_4$alkyl or $C_1$-$C_4$alkoxy ; $C_2$-$C_4$alkanoyloxymethyl or $C_2$-$C_4$alkanoyloxymethyl substituted on oxymethyl by $C_1$-$C_4$alkyl or cycloalkyl, $R^1$ represents carboxy, $C_1$-$C_4$alkoxycarbonyl, carbamoyl or N-mono- or N,N-di-$C_1$-$C_4$alkylcarbamyl, $R^2$ represents hydrogen, $C_1$-$C_4$alkyl, aryl-$C_1$-$C_4$alkyl, $C_2$-$C_7$alkanoyl, benzoyl, $C_1$-$C_4$alkoxycarbonyl, phenylacetyl, 3-phenylpropionyl or α-amino-$C_2$-$C_4$alkanoyl, $R^4$ represents hydrogen, lower alkyl, lower alkoxy, halogen or trifluoromethyl and A represents $C_1$-$C_4$alkylene ; or a pharmaceutically acceptable salt thereof.

11. A compound according to claim 10 of the formula V

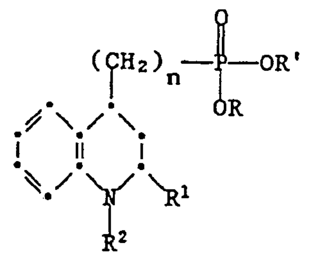

(V)

or a perhydroquinoline derivative thereof wherein n represents the integer 1, 2 or 3 ; R and R′ independently represent hydrogen, $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, $R^1$ represents carboxy, $C_1$-$C_4$alkoxycarbonyl or carbamoyl, $R^2$ represents hydrogen, $C_1$-$C_4$alkyl, $C_2$-$C_4$alkanoyl, benzoyl or benzoyl substituted by $C_1$-$C_4$alkyl, by $C_1$-$C_4$alkoxy, by halogen or by trifluoromethyl ; or a pharmaceutically acceptable salt of said compound having a salt-forming functional grouping.

12. A compound of formula V according to claim 11 wherein n represents the integer 1, 2 or 3 ; R and R′ represent hydrogen, $R^1$ represents carboxy or $C_1$-$C_4$alkoxycarbonyl and $R^2$ represents hydrogen ; or a pharmaceutically acceptable salt thereof.

13. A compound according to claim 12 being cis-4-phosphonomethyl-1,2,3,4-tetrahydroquinoline-2-carboxylic acid or a pharmaceutically acceptable salt thereof.

14. A compound according to claim 1 of the formula VI

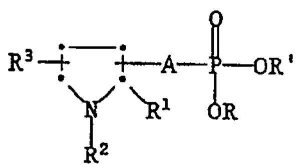

(VI)

or a compound of formula VI with a double bond present between C-3 and C-4 of the pyrrolidinyl ring, wherein R and R′ independently represent hydrogen, $C_1$-$C_4$alkyl, benzyl, benzyl substituted on phenyl by halogen, $C_1$-$C_4$alkyl or $C_1$-$C_4$alkoxy ; $C_2$-$C_4$alkanoyloxymethyl or $C_2$-$C_4$alkanoyloxy-methyl substituted on oxymethyl by $C_1$-$C_4$alkyl or cycloalkyl, $R^1$ represents carboxy, $C_1$-$C_4$alkoxycarbonyl, carbamoyl or N-mono- or N,N-di-$C_1$-$C_4$ alkylcarbamoyl, $R^2$ represents hydrogen, $C_1$-$C_4$alkyl, $C_2$-$C_7$alkanoyl, benzoyl, $C_1$-$C_4$alkoxycarbonyl, phenylacetyl, 3-phenylpropionyl or α-amino-$C_2$-$C_4$alkanoyl, $R^3$ represents hydrogen, $C_1$-$C_4$alkyl or aryl-$C_1$-$C_4$alkyl and A represents $C_1$-$C_4$alkylene ; or a salt thereof.

15. A compound of formula VI according to claim 14 wherein the phosphono bearing group is attached at the 3- or 4-position, R and R′ independently represent hydrogen or $C_2$-$C_4$alkanoyloxymethyl, $R^1$ represents carboxy, $C_1$-$C_4$alkoxycarbonyl or carbamoyl, $R^2$ and $R^3$ represent hydrogen and A represents $C_1$-$C_3$alkylene ; or a pharmaceutically acceptable salt thereof.

16. A compound according to claim 15 being 4-phosphonomethylpyrrolidine-2-carboxylic acid or a pharmaceutically acceptable salt thereof.

17. A compound according to any one of claims 2 to 5, 7 to 9, 11 to 13, 15 and 16 for use in the therapeutic treatment of the human or animal body.

18. A compound according to any one of claims 1, 6, 10 and 14 for use in the therapeutic treatment of the human or animal body.

19. A pharmaceutical composition comprising a compound claimed in any one of claims 2 to 5, 7 to 9, 11 to 13 and 17 in admixture or conjunction with one or more pharmaceutically suitable carriers.

20. A pharmaceutical composition comprising a compound claimed in any one of claims 1, 6, 10, 14 and 18 in admixture or conjunction with one or more pharmaceutically suitable carriers.

21. A compound claimed in any one of claims 1 to 16 as an antagonist of the N-methyl-D-aspartate (NMDA) sensitive excitatory amino acid receptor.

22. Use of a compound claimed in any one of claims 1 to 16 for the manufacture of a pharmaceutical composition.

23. Use of a compound claimed in any one of claims 1 to 16 for the manufacture of pharmaceutical composition for treatment of diseases responsive to blockade of the N-methyl-D-aspartate (NMDA) sensitive excitatory amino acid receptor.

24. Process for the manufacture of a compound of formula I as claimed in claim 1, or of a salt thereof, which

consists in

a) reducing a compound of the formula VII

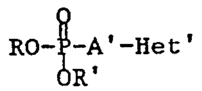 (VII)

said compound of formula VII being otherwise identical to a compound of formula I but having one or more double bonds present in the five- or six-membered heterocyclic ring Het' and/or within A' which represents A or lower alkenylene or lower alkanylidene with the double bond attached to the heterocyclic ring ; or

b) condensing a compound of the formula VIII

X-A-Het (VIII)

wherein Het and A are as defined for formula I and X represents reactive esterified hydroxy, with a compound capable of introducing the phosphonic acid moiety, having one of formulae IX or X,

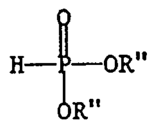 (IX) $P(R''')_3$ . (X)

wherein R″ represents lower alkyl and R‴ represents halogen or lower alkoxy and, if required, converting the resulting phosphonic acid derivative to the phosphonic acid or other ester derivative thereof ; or

c) converting to $R^1$ a substituent other than $R^1$ at position 2 of the heterocyclic ring in a compound otherwise identical to a compound of the invention ; and carrying out the said processes while, if necessary, temporarily protecting any interfering reactive group(s) in these processes, and then liberating the resulting compound of the invention ; and, if desired, converting a resulting compound of the invention into another compound of the invention, and/or, if desired, converting a resulting free compound into a salt or a resulting salt into the free compound or into another salt ; and/or separating a mixture of isomers or racemates obtained into the single isomers or racemates ; and/or, if desired, resolving a racemate obtained into the optical antipodes.

**Claims for the Contracting State : AT**

1. A compound of the formula I,

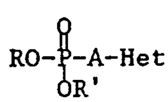 (I),

wherein R and R′ independently represent hydrogen, $C_1$-$C_4$alkyl, benzyl, benzyl substituted on phenyl by halogen, $C_1$-$C_4$alkyl or $C_1$-$C_4$alkoxy, $C_2$-$C_7$alkanoyloxymethyl or $C_2$-$C_7$alkanoyloxymethyl substituted on oxymethyl by $C_1$-$C_4$alkyl or $C_3$-$C_8$cycloalkyl, A denotes $C_1$-$C_4$alkylene and Het represents a 2-$R^1$-pyrrolidinyl, 2-$R^1$-2,5-dihydropyrrolyl, 2-$R^1$-1,2,3,6-tetrahydropyridinyl, 2-$R^1$-1,2,5,6-tetrahydropyridinyl, 2-$R^1$-piperidinyl, 2-$R^1$-tetrahydroquinolinyl, 2-$R^1$-perhydroquinolinyl, 2-$R^1$-carboxy-2,3-di-hydroindolyl or 2-$R^1$-perhydroindolyl group which may be additionally N-substituted by $C_1$-$C_4$alkyl, phenyl-$C_1$-$C_4$alkyl, $C_2$-$C_7$alkanoyl, benzoyl, $C_1$-$C_4$alkoxycarbonyl, benzyloxycarbonyl, phenylacetyl, 3-phenylpropionyl or α-amino-$C_2$-$C_4$alkyl, C-substituted in the heterocyclic 5- or 6-ring by $C_1$-$C_4$alkyl or phenyl-$C_1$-$C_4$alkyl and/or, if present, in the benzo or cyclohexano ring by $C_1$-$C_4$alkyl, $C_1$-$C_4$alkoxy, halo or trifluoromethyl, or of a salt thereof, which consists in

a) reducing a compound of the formula VII

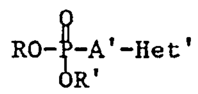

(VII)

said compound of formula VII being otherwise identical to a compound of formula I but having one or more double bonds present in the five- or six-membered heterocyclic ring Het' and/or within A' which represents A or lower alkenylene or lower alkanylidene with the double bond attached to the heterocyclic ring ; or
b) condensing a compound of the formula VIII

X-A-Het (VIII)

wherein Het and A are as defined for formula I and X represents reactive esterified hydroxy, with a compound capable of introducing the phosphonic acid moiety, having one of formulae IX or X,

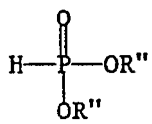

(IX) $P(R''')_3$ (X)

wherein R'' represents lower alkyl and R''' represents halogen or lower alkoxy and, if required, converting the resulting phosphonic acid derivative to the phosphonic acid or other ester derivative thereof ; or
c) converting to $R^1$ a substituent other than $R^1$ at position 2 of the heterocyclic ring in a compound otherwise identical to a compound of the invention ; and carrying out the said processes while, if necessary, temporarily protecting any interfering reactive group(s) in these processes, and then liberating the resulting compound of the invention ; and, if desired, converting a resulting compound of the invention into another compound of the invention, and/or, if desired, converting a resulting free compound into a salt or a resulting salt into the free compound or into another salt ; and/or separating a mixture of isomers or racemates obtained into the single isomers or racemates ; and/or, if desired, resolving a racemate obtained into the optical antipodes.
2. Process according to claim 1 for the manufacture of a compound of the formula II

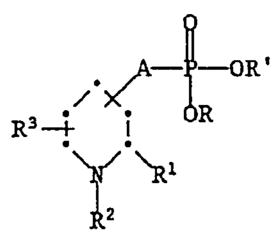

(II)

or a compound of formula II with a double bond present between C-3 and C-4 or between C-4 and C-5 of the piperidinyl ring, in which the phosphono bearing chain is attached at the 3-, 4-, or 5-position of the piperidinyl or tetrahydropyridinyl ring, and wherein R and R' independently represent hydrogen, $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, cyclohexyl or cyclopentyl, $R^1$ represents carboxy, carbamoyl, N-mono- or N,N-di-$C_1$-$C_4$alkylcarbamoyl or $C_1$-$C_4$alkoxycarbonyl, $R^2$ and $R^3$ represent hydrogen or $C_1$-$C_4$alkyl and A represents $C_1$-$C_4$alkylene ; or of a pharmaceutically acceptable salt thereof.

3. Process according to claim 1 for the manufacture of a compound of formula II according to claim 2 wherein R and R' independently represent hydrogen, $C_2$-$C_4$alkanoyloxymethyl or $C_2$-$C_4$alkanoyloxymethyl substituted on oxymethyl by $C_1$-$C_4$alkyl, $R^1$ represents carboxy, carbamoyl or $C_1$-$C_4$alkoxycarbonyl, $R^2$ and $R^3$ represent hydrogen and A is at the 4-position and represents $C_1$-$C_4$alkylene ; or of a pharmaceutically acceptable salt thereof.

4. Process according to claim 1 for the manufacture of a compound of the formula III

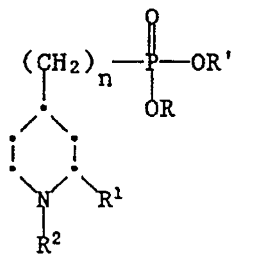

(III)

wherein n represents the integer 1, 2 or 3 ; R and R′ independently represent hydrogen, $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, $R^1$ represents carboxy, $C_1$-$C_4$alkoxycarbonyl or carbamoyl and $R^2$ represents hydrogen, $C_1$-$C_4$alkyl, $C_2$-$C_4$alkanoyl or benzoyl ; or of a pharmaceutically acceptable salt thereof.

5. Process according to claim 1 for the manufacture of a compound of formula III according to claim 4 wherein n represents the integer 1, 2 or 3 ; R and R′ both represent hydrogen or $C_2$-$C_4$alkanoyloxymethyl ; or one of R and R′ represents hydrogen and the other of R and R′ represents $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, $R^1$ represents carboxy or $C_1$-$C_4$alkoxy-carbonyl and $R^2$ represents hydrogen ; or of a pharmaceutically acceptable salt thereof.

6. Process according to claim 1 for the manufacture of a compound of formula III according to claim 4 wherein n represents the integer 1, 2 or 3 ; R and R′ represent hydrogen, $R^1$ represents carboxy or $C_1$-$C_4$alkoxycarbonyl and $R^2$ represents hydrogen ; or of a pharmaceutically acceptable salt thereof.

7. Process according to claim 1 for the manufacture of cis-4-phosphonomethyl-2-piperidinecarboxylic acid or of a pharmaceutically acceptable salt thereof.

8. Process according to claim 1 for the manufacture of cis-4-(3-phosphonopropyl)-2-piperidinecarboxylic acid or of a pharmaceutically acceptable salt thereof.

9. Process according to claim 1 for the manufacture of cis-4-phosphonomethyl-2-piperidinecarboxylic acid ethyl ester or of a pharmaceutically acceptable salt thereof.

10. Process according to claim 1 for the manufacture of a compound of the formula IV

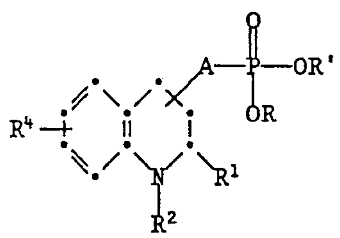

(IV)

or a perhydro derivative thereof, wherein R and R′ independently represent hydrogen, $C_1$-$C_4$alkyl, benzyl, benzyl substituted on phenyl by halogen, $C_1$-$C_4$alkyl or $C_1$-$C_4$alkoxy ; $C_2$-$C_4$alkanoyloxymethyl or $C_2$-$C_4$alkanoyloxymethyl substituted on oxymethyl by $C_1$-$C_4$alkyl or cycloalkyl, $R^1$ represents carboxy, $C_1$-$C_4$alkoxycarbonyl, carbamoyl or N-mono- or N,N-di-$C_1$-$C_4$alkylcarbamyl, $R^2$ represents hydrogen, $C_1$-$C_4$alkyl, aryl-$C_1$-$C_4$alkyl, $C_2$-$C_7$alkanoyl, benzoyl, $C_1$-$C_4$alkoxycarbonyl, phenylacetyl, 3-phenylpropionyl or α-amino-$C_2$-$C_4$alkanoyl, $R^4$ represents hydrogen, lower alkyl, lower alkoxy, halogen or trifluoromethyl and A represents $C_1$-$C_4$alkylene ; or of a pharmaceutically acceptable salt thereof.

11. Process according to claim 1 for the manufacture of a compound of the formula V

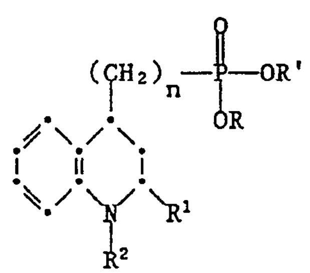

(V)

or a perhydroquinoline derivative thereof wherein n represents the integer 1, 2 or 3 ; R and R′ independently

represent hydrogen, $C_1$-$C_4$alkyl, benzyl, $C_2$-$C_4$alkanoyloxymethyl or $C_2$-$C_4$alkanoyloxymethyl substituted on oxymethyl by $C_1$-$C_4$alkyl, $R^1$ represents carboxy, $C_1$-$C_4$alkoxycarbonyl or carbamoyl, $R^2$ represents hydrogen, $C_1$-$C_4$alkyl, $C_2$-$C_4$alkanoyl, benzoyl or benzoyl substituted by $C_1$-$C_4$alkyl, by $C_1$-$C_4$alkoxy, by halogen or by trifluoromethyl ; or of a pharmaceutically acceptable salt thereof.

12. Process according to claim 1 for the manufacture of a compound of formula V according to claim 11 wherein n represents the integer 1, 2 or 3 ; R and R′ represent hydrogen, $R^1$ represents carboxy or $C_1$-$C_4$alkoxy-carbonyl and $R^2$ represents hydrogen ; or of a pharmaceutically acceptable salt thereof.

13. Process according to claim 1 for the manufacture of cis-4-phosphonomethyl-1,2,3,4-tetrahydroquinoline-2-carboxylic acid or of a pharmaceutically acceptable salt thereof.

14. Process according to claim 1 for the manufacture of a compound of the formula VI

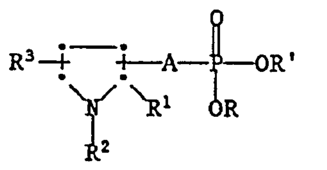

(VI)

or a compound of formula VI with a double bond present between C-3 and C-4 of the pyrrolidinyl ring, wherein R and R′ independently represent hydrogen, $C_1$-$C_4$alkyl, benzyl, benzyl substituted on phenyl by halogen, $C_1$-$C_4$alkyl or $C_1$-$C_4$alkoxy ; $C_2$-$C_4$alkanoyloxymethyl or $C_2$-$C_4$alkanoyloxy-methyl substituted on oxymethyl by $C_1$-$C_4$alkyl or cycloalkyl, $R^1$ represents carboxy, $C_1$-$C_4$alkoxycarbonyl, carbamoyl or N-mono- or N,N-di-$C_1$-$C_4$alkylcarbamoyl, $R^2$ represents hydrogen, $C_1$-$C_4$alkyl, $C_2$-$C_7$alkanoyl, benzoyl, $C_1$-$C_4$alkoxycarbonyl, phenylacetyl, 3-phenylpropionyl or α-amino-$C_2$-$C_4$alkanoyl, $R^3$ represents hydrogen, $C_1$-$C_4$alkyl or aryl-$C_1$-$C_4$alkyl and A represents $C_1$-$C_4$alkylene ; or of a pharmaceutically acceptable salt thereof.

15. Process according to claim 1 for the manufacture of a compound of formula VI according to claim 14 wherein the phosphono bearing group is attached at the 3- or 4-position, R and R′ independently represent hydrogen or $C_2$-$C_4$alkanoyloxymethyl, $R^1$ represents carboxy, $C_1$-$C_4$alkoxy-carbonyl or carbamoyl, $R^2$ and $R^3$ represent hydrogen and A represents $C_1$-$C_3$alkylene ; or of a pharmaceutically acceptable salt thereof.

16. Process according to claim 1 for the manufacture of 4-phosphono-methylpyrrolidine-2-carboxylic acid or of a pharmaceutically acceptable salt thereof.

17. Process for the manufacture of pharmaceutical composition wherein a compound obtainable according to any one of claims 1 to 16 is admixed to one or more pharmaceutically acceptable carriers.

## Ansprüche

**Patentansprüche für BE, CM, DE, FR, GB, IT, LI, LU, NL, SE**

1. Verbindung der formel I

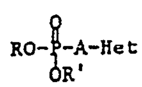

(I),

worin R und R′ unabhängig Wasserstoff, $C_1$-$C_4$-Alkyl, Benzyl, Benzyl, das am Phenyl substituiert ist durch Halogen, $C_1$-$C_4$-Alkyl oder $C_1$-$C_4$-Alkoxy, $C_2$-$C_7$-Alkanoyloxymethyl oder $C_2$-$C_7$-Alkanoyloxymethyl, das am Oxymethyl substituiert ist durch $C_1$-$C_4$-Alkyl oder $C_3$-$C_8$-Cycloalkyl, darstellen, A $C_1$-$C_4$-Alkylen bezeichnet und Het eine 2-$R^1$-Pyrrolidinyl-, 2-$R^1$-2,5-Dihydropyrrolyl-, 2-$R^1$-1,2,3,6-Tetrahydropyridinyl-, 2-$R^1$-1,2,5,6-Tetrahydropyridinyl-, 2-$R^1$-Piperidinyl-, 2-$R^1$-Tetrahydrochinolinyl-, 2-$R^1$-Perhydrochinolinyl-, 2-$R^1$-Carboxy-2,3-dihydroindolyl- oder 2-$R^1$-Perhydroindolyl-Gruppe darstellt, die zusätzlich N-substituiert sein kann durch $C_1$-$C_4$-Alkyl, Phenyl-$C_1$-$C_4$-alkyl, $C_2C_7$-Alkanoyl, Benzoyl, $C_1$-$C_4$-Alkoxycarbonyl, Benzyloxycarbonyl, Phenylacetyl, 3-Phenylpropionyl oder α-Amino-$C_2$-$C_4$-alkyl, C-substituiert im heterocyclischen 5- oder 6-Ring durch $C_1$-$C_4$-Alkyl oder Phenyl-$C_1$-$C_4$-alkyl und/oder, falls vorhanden, im Benzo- oder Cyclohexanoring durch $C_1$-$C_4$-Alkyl, $C_1$-$C_4$-Alkoxy, Halo oder Trifluormethyl, oder ein Salz davon.

2. Verbindung nach Anspruch 1 mit der formel II

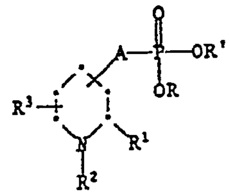

(II)

oder eine Verbindung der formel II mit einer Doppelbindung zwischen C-3 und C-4 oder zwischen C-4 und C-5 des Piperidinylringes, worin die Phosphono-tragende Kette an der 3-, 4- oder 5-Position des Piperidinyl- oder Tetrahydropyridinylringes gebunden ist, und worin R und R′ unabhängig Wasserstoff, $C_1$-$C_4$-Alkyl, Benzyl, $C_2$-$C_4$-Alkanoyloxymethyl oder $C_2$-$C_4$-Alkanoyloxymethyl, das am Oxymethyl substituiert ist durch $C_1$-$C_4$-Alkyl, Cyclohexyl oder Cyclopentyl, darstellen, $R^1$ Carboxy, Carbamoyl, N-Mono- oder N,N-Di-$C_1$-$C_4$-alkylcarbamoyl oder $C_1$-$C_4$-Alkoxycarbonyl darstellt, $R^2$ und $R^3$ Wasserstoff oder $C_1$-$C_4$-Alkyl darstellen und A für $C_1$-$C_4$-Alkylen steht ; oder ein pharmazeutisch annehmbares Salz davon.

3. Verbindung der formel II nach Anspruch 2, worin R und R′ unabhängig Wasserstoff, $C_2$-$C_4$-Alkanoyloxymethyl oder am Oxymethyl durch $C_1$-$C_4$-Alkyl substituiertes $C_2$-$C_4$-Alkanoyloxymethyl darstellen, $R^1$ Carboxy, Carbamoyl oder $C_1$-$C_4$-Alkorycarbonyl darstellt, $R^2$ und $R^3$ Wasserstoff darstellen und A an der 4-Position ist und $C_1$-$C_4$-Alkylen darstellt ; oder ein pharmazeutisch annehmbares Salz davon.

4. Verbindung nach Anspruch 2 mit der Formel III

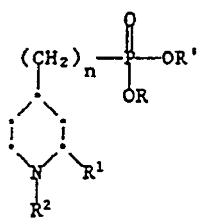

(III)

worin n die ganze Zahl 1, 2 oder 3 darstellt ; R und R′ unabhängig Wasserstoff, $C_1$-$C_4$-Alkyl, Benzyl, $C_2$-$C_4$-Alkanoyloxymethyl oder am Oxymethyl durch $C_1$-$C_4$-Alkyl substituiertes $C_2$-$C_4$-Alkanoyloxymethyl darstellen, $R^1$ Carboxy, $C_1$-$C_4$ Alkoxycarbonyl oder Carbamoyl darstellt und $R^2$ Wasserstoff, $C_1$-$C_4$-Alkyl, $C_2$-$C_4$-Alkanoyl oder Benzoyl darstellt ; oder ein pharmazeutisch annehmbares Salz bzsagter Verbindung, die eine salzbildzndе funktionelle Gruppe hat.

5. Verbindung der Formel III nach Anspruch 4, worin n die ganze Zahl 1, 2 oder 3 ist ; R und R′ beide Wasserstoff oder $C_2$-$C_4$-Alkanoyloxymethyl sind ; oder eines von R und R′ Wasserstoff ist und das andere von R und R′ $C_1$-$C_4$-Alkyl, Benzyl, $C_2$-$C_4$-Alkanoyloxymethyl oder am Oxymethyl durch $C_1$-$C_4$-Alkyl substituiertes $C_2$-$C_4$-Alkanoylorymethyl ist, $R^1$ Carboxy oder $C_1$-$C_4$-Alkoxycarbonyl darstellt und $R^2$ Wasserstoff ist ; oder ein pharmazeutisch annehmbares Salz davon.

6. Verbindung der Formel III nach Anspruch 4, worin n die ganze Zahl 1, 2 oder 3 ist ; R und R′ Wasszrstoff sind ; $R^1$ Carboxy oder $C_1$-$C_4$-Alkoxycarbonyl ist und $R^2$ Wasserstoff darstellt ; oder ein pharmazeutisch annehmbares Salz davon.

7. Verbindung nach Anspruch 6, die cis-4-Phosphonomethyl-2-piperidincarbonsäure oder ein pharmazeutisch annehmbares Salz davon ist.

8. Verbindung nach Anspruch 6, die cis-4-(3-Phosphonopropyl)-2-piperidincarbonsäure oder ein pharmazeutisch annehmbares Salz davon ist.

9. Verbindung nach Anspruch 6, die cis-4-Phosphonomethyl-2-piperidincarbonsäure-ethylester oder ein pharmazeutisch annehmbares Salz davon ist.

10. Verbindung nach Anspruch 1 mit der Formel IV

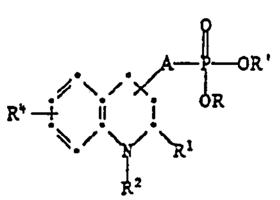

(IV)

oder ein Perhydro-Derivat davon, worin R und R′ unabhängig Wasserstoff, $C_1$-$C_4$-Alkyl, Benzyl, am Phenyl durch Halogen, $C_1$-$C_4$-Alkyl oder $C_1$-$C_4$-Alkoxy substituiertes Benzyl : $C_2$-$C_4$-Alkanoyloxymethyl oder am Oxymethyl durch $C_1$-$C_4$-Alkyl oder Cycloakyl substituiertes $C_2$-$C_4$-Alkanoyloxymethyl darstellen, $R^1$ Carboxy, $C_1$-$C_4$-Alkoxycarbonyl, Carbamoyl oder M-Mono- oder N,N-Di-$C_1$-$C_4$-alkylcarbamyl ist, $R^2$ Wasserstoff, $C_1$-$C_4$-Alkyl, Aryl-$C_1$-$C_4$-alkyl, $C_2$-$C_7$-Alkanoyl, Benzoyl, $C_1$-$C_4$-Alkoxycarbonyl, Phenylacetyl, 3-Phenylpropionyl oder α-Amino-$C_2$-$C_4$-alkanoyl ist, $R^4$ Wasserstoff, niederes Alkyl, niederes Alkoxy, Halogen oder Trifluormethyl ist und A für $C_1$-$C_4$-Alkylen steht ; oder ein pharmazeutisch annehmbares Salz davon.

11. Verbindung nach Anspruch 10 mit der formel V

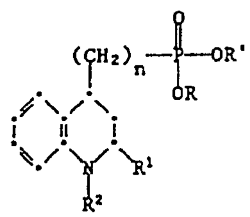

(V)

oder ein Perhydrochinolin-Derivat davon, worin n die ganze Zahl 1, 2 oder 3 ist ; R und R′ unabhängig Wasserstoff, $C_1$-$C_4$-Alkyl, Benzyl, $C_2$-$C_4$-Alkanoyloxymethyl oder am Oxymethyl durch $C_1$-$C_4$-Alkyl substituiertes $C_2$-$C_4$-Alkanoyloxymethyl ist, $R^1$ Carboxy, $C_1$-$C_4$-Alkoxycarbonyl oder Carbamoyl ist, $R^2$ Wasserstoff, $C_1$-$C_4$-Alkyl, $C_2$-$C_4$-Alkanoyl, Benzoyl oder durch $C_1$-$C_4$-Alkyl, $C_1$-$C_4$-Alkoxy, Halogen oder Trifluormethyl substituiertes Benzoyl ist ; oder ein pharmazeutisch annehmbares Salz dieser verbindung, die eine salzbildende funktionelle Gruppe hat.

12. Verbindung der formel V nach Anspruch 11, worin n die ganze Zahl 1, 2 oder 3 darstellt ; R und R′ Wasserstoff darstellen ; $R^1$ Carboxy oder $C_1$-$C_4$-Alkoxycarbonyl ist und $R^2$ Wasserstoff darstellt ; oder ein pharmazeutisch annehmbares Salz davon.

13. Verbindung nach Anspruch 12, die cis-4-Phosphonomethyl-1,2,3,4-tetrahydrochinolin-2-carbonsäure oder ein pharmazeutisch annehmbares Salz davon ist.

14. Verbindung nach Anspruch 1 mit der formel VI

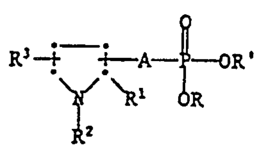

(VI)

oder eine verbindung der formel VI mit einer Doppelbindung zwischen C-3 und C-4 des Pynolidinylringes, worin R und R′ unabhängig für Wasserstoff, $C_1$-$C_4$-Alkyl, Benzyl, am Phenyl durch Halogen, $C_1$-$C_4$-Alkyl oder $C_1$-$C_4$-Alkoxy substituiertes Benzyl ; $C_2$-$C_4$-Alkanoyloxymethyl oder am Oxymethyl durch $C_1$-$C_4$-Alkyl oder Cycloalkyl substituiertes $C_2$-$C_4$-Alkanoyloxymethyl ist, $R^1$ Carboxy, $C_1$-$C_4$ Alkoxycarbonyl, Carbamoyl oder N-Mono- oder N,N-Di-$C_1$-$C_4$-alkylcarbamoyl ist, $R^2$ Wasserstoff, $C_1$-$C_4$-Alkyl, $C_2$-$C_7$-Alkanoyl, Benzoyl, $C_1$-$C_4$-Alkoxycarbonyl, Phenylacetyl, 3-Phenylpropionyl oder α-Amino-$C_2$-$C_4$-alkanoyl ist, $R^3$ Wasserstoff, $C_1$-$C_4$-Alkyl oder Aryl-$C_1$-$C_4$-alkyl ist und A $C_1$-$C_4$-Alkylen darstellt ; oder ein Salz davon.

15. Verbindung der formel VI nach Anspruch 14, worin die Phosphono-tragende Gruppe an der 3- oder 4-Position gebunden ist, R und R′ unabhängig Wasserstoff oder $C_2$-$C_4$-Alkanoyloxymethyl sind, $R^1$ Carboxy, $C_1$-$C_4$-Alkoxycarbonyl oder Carbamoyl ist, $R^2$ und $R^3$ Wasserstoff sind und A $C_1$-$C_4$-Alkylen ist ; oder ein pharmazeutisch annehmbares Salz davon.

16. Verbindung nach Anspruch 15, die 4-Phosphonomethylpyrrolidin-2-carbonsäure oder ein pharmazeutisch annehmbares Salz davon ist.

17. Verbindung nach irgendeinem der Ansprüche 2 bis 5, 7 bis 9, 11 bis 13, 15 und 16 zur Verwendung bei der therapeutischen Behandlung des menschlichen oder tierischen Körpers.

18. Verbindung nach irgendeinem der Ansprüche 1, 6, 10 und 14 zur Verwendung bei der therapeutischen Behandlung des menschlichen oder tierischen Körpers.

19. Pharmazeutische Zusammensetzung, die eine in irgendeinem der Ansprüche 2 bis 5, 7 bis 9, 11 bis 13 und 17 beanspruchte Verbindung unter Beifügung oder Zumischung von einem oder mehreren pharmazeutisch geeigneten Trägern enthält.

20. Pharmazeutische Zusammensetzung, die eine in irgendeinem der Ansprüche 1, 6, 10, 14 und 18 beanspruchte Verbindung unter Beifügung oder Zumischung von einem oder mehreren pharmazeutisch geeigneten Trägern enthält.

21. Verbindung, die in irgendeinem der Ansprüche 1 bis 16 beansprucht wird, als Antagonist des N-Methyl-D-aspartat (NMDA)-empfindlichen Aminosäurereizrezeptors.

22. Verwendung einer in irgendeinem der Ansprüche 1 bis 16 beanspruchten Verbindung zur Herstellung einer pharmazeutischen Zusammensetzung.

23. Verwendung einer in irgendeinem der Ansprüche 1 bis 16 beanspruchten Verbindung zur Herstellung einer pharmazeutischen Zusammensetzung zur Behandlung von Erkrankungen, die auf das Blockieren des N-Methyl-D-aspartat (NMDA)-empfindlichen Aminosäurereizrezeptors ansprechen.

24. Verfahren zur Herstellung einer Verbindung der Formel I, wie im Anspruch 1 beansprucht, oder eines Salzes davon, welches Verfahren umfaßt :

a) Reduktion einer Verbindung mit der Formel VII

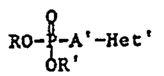 (VII)

wobei benannte Verbindung von Formel VII sonst identisch mit einer Verbindung der Formel I ist, jedoch mit einer oder mehreren Doppelbindungen in dem heterocyclischen Fünf- oder Sechsring Het′ und/oder innerhalb von A′, das A oder ein niederes Alkenylen oder niederes Alkanyliden darstellt, wo die Doppelbindung an dem heterocyclischen Ring gebunden ist ; oder

b) Kondensation einer Verbindung der Formel VIII

X-A–Het (VIII)

worin Het und A wie für Formel I definiert sind und X ein reaktionsfähiges verestertes Hydroxy bezeichnet, mit einer Verbindung, durch die die Phosphonsäureeinheit eingeführt werden kann, die eine der Formeln IX oder X aufweist

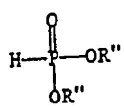 (IX) $P(R''')_3$ (X)

worin R″ niederes Alkyl bezeichnet und R‴ ein Halogen oder niederes Alkoxy ist, und erforderlichenfalls Umwandlung des entstehenden Phosphonsäurederivats in die Phosphonsäure oder andere Esterderivate davon ; oder

c) Umwandlung zu $R^1$ eines anderen Substituenten als $R^1$ in Stellung 2 des heterocyclischen Ringes in einer Verbindung, die sonst mit einer erfindungsgemäßen Verbindung identisch ist ; und Durchführung der benannten Verfahren erforderlichenfalls bei vorübergehendem Schutz aller störenden reaktionsfähigen Gruppen in diesen Verfahren, und anschließend Freisetzung der entstehenden erfindungsgemäßen Verbindung ; und gewünschtenfalls Umwandlung einer entstehenden erfindungsgemäßen Verbindung in eine andere Verbindung der Erfindung und/oder gewünschtenfalls Umwandlung einer entstehenden freien Verbindung in ein Salz oder eines entstehenden Salzes in die freie Verbindung oder ein anderes Salz ; und/oder Trennen eines erhaltenen Gemisches von Isomeren oder Racematen in die einzelnen Isomere oder Racemate ; und/oder gewünschtenfalls Auflösung eines entstandenen Racemats in die optischen Antipoden.

**Patentansprüche für AT**

1. Verfahren zur Herstellung einer verbindung der Formel I :

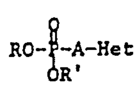 (I),

worin R und R′ unabhängig Wasserstoff, $C_1$-$C_4$-Alkyl, Benzyl, Benzyl, das am Phenyl substituiert ist durch Halogen, $C_1$-$C_4$-Alkyl oder $C_1$-$C_4$-Alkoxy, $C_2$-$C_7$-Alkanoyloxymethyl oder $C_2$-$C_7$-Alkanoyloxymethyl, das am Oxymethyl substituiert ist durch $C_1$-$C_4$-Alkyl oder $C_3$-$C_8$-Cycloalkyl, darstellen, A $C_1$-$C_4$-Alkylen bezeichnet und Het

eine 2-$R^1$-Pyrrolidinyl-, 2-$R^1$-2,5-Dihydropyrrolyl-, 2-$R^1$-1,2,3,6-Tetrahydropyridinyl-, 2-$R^1$-1,2,5,6-Tetrahydropyridinyl-, 2-$R^1$-Piperidinyl-, 2-$R^1$-Tetrahydrochinolinyl-, 2-$R^1$-Perhydrochinolinyl-, 2-$R^1$-Carboxy-2,3-dihydroindolyl- oder 2-$R^1$-Perhydroindolyl-Gruppe darstellt, die zusätzlich N-substituiert sein kann durch $C_1$-$C_4$-Alkyl, Phenyl-$C_1$-$C_4$-alkyl, $C_2$-$C_7$-Alkanoyl, Benzoyl, $C_1$-$C_4$-Alkoxycarbonyl, Benzyloxycarbonyl, Phenylacetyl, 3-Phenylpropionyl oder α-Amino-$C_2$-$C_4$-alkyl, C-substituiert im heterocyclischen 5- oder 6-Ring durch $C_1$-$C_4$-Alkyl oder Phenyl-$C_1$-$C_4$-alkyl und/oder, falls vorhanden, im Benzo- oder Cyclohexanoring durch $C_1$-$C_4$-Alkyl, $C_1$-$C_4$-Alkoxy, Halo oder Trifluormethyl, oder eines Salzes davon, welches verfahren umfaßt :

a) Reduktion einer verbindung mit der Formel VII

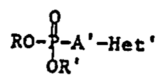

(VII)

wobei benannte verbindung von Formel VII sonst identisch mit einer verbindung der Formel I ist, jedoch mit einer oder mehreren Doppelbindungen in dem heterocyclischen Fünf- oder Sechsring Het′ und/oder innerhalb von A′, das A oder ein niederes Alkenylen oder niederes Alkanyliden darstellt, wo die Doppelbindung an den heterocyclischen Ring gebunden ist ; oder

b) Kondensation einer verbindung der Formel

X-A-Het (VIII)

worin Het und A wie für Formel I definiert sind und X ein reaktionsfähiges verestertes Hydroxy bezeichnet, mit einer verbindung, durch die die Phosphonsäureeinheit eingeführt werden kann, die eine der Formeln IX oder X aufweist

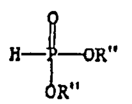

(IX) $P(R''')_3$ (X)

worin R″ niederes Alkyl bezeichnet und R‴ ein Halogen oder niederes Alkoxy ist,und erforderlichenfalls Umwandlung des entstehenden Phosphonsäurederivats in die Phosphonsäure oder andere Esterderivate davon ; oder

c) Umwandlung zu $R^1$ eines anderen Substituenten als $R^1$ in Stellung 2 des heterocyclischen Ringes in einer Verbindung, die sonst mit einer erfindungsgemäßen Verbindung identisch ist ; und Durchführung der benannten Verfahren erforderlichenfalls bei vorübergehendem Schutz aller störenden reaktionsfähigen Gruppen in diesen Verfahren, und anschließend Freisetzung der entstehenden erfindungsgemäßen Verbindung ; und gewünschtenfalls Umwandlung einer entstehenden erfindungsgemäßen Verbindung in eine andere Verbindung der Erfindung und/oder gewünschtenfalls Umwandlung einer entstehenden freien Verbindung in ein Salz oder eines entstehenden Salzes in die freie Verbindung oder ein anderes Salz ; und/oder Trennen eines erhaltenen Gemisches von Isomeren oder Racematen in die einzelnen Isomere oder Racemate ; und/oder gewünschtenfalls Auflösung eines entstandenen Racemats in die optischen Antipoden.

2. Verfahren nach Anspruch 1 zur Herstellung einer Verbindung der Formel II

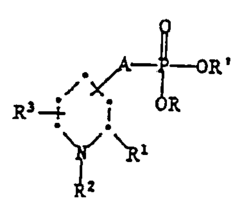

(II)

oder einer Verbindung der Formel II mit einer Doppelbindung zwischen C-3 und C-4 oder zwischen C-4 und C-5 des Piperidinylringes, worin die Phosphono-tragende Kette an der 3-, 4- oder 5-Position des Piperidinyl- oder Tetrahydropyridinylringes gebunden ist, und worin R und R′ unabhängig Wasserstoff, $C_1$-$C_4$-Alkyl, Benzyl, $C_2$-$C_4$-Alkanoyloxymethyl oder $C_2$-$C_4$-Alkanoyloxymethyl, das am Oxymethyl substituiert ist durch $C_1$-$C_4$-Alkyl, Cyclohexyl oder Cyclopentyl, darstellen, $R^1$ Carboxy, Carbamoyl, N-Mono- oder N,N-Di-$C_1$-$C_4$-Alkylcarbamoyl oder $C_1$-$C_4$-Alkoxycarbonyl darstellt, $R^2$ und $R^3$ Wasserstoff oder $C_1$-$C_4$-Alkyl darstellen und à für $C_1$-$C_4$-Alkylen

steht ; oder eines pharmazeutisch annehmbaren Salzes davon.

3. Verfahren nach Anspruch 1 zur Herstellung einer Verbindung der Formel II nach Anspruch 2, worin R und R' unabhängig Wasserstoff, $C_2$-$C_4$-Alkanoyloxymethyl oder am Oxymethyl durch $C_1$-$C_4$-Alkyl substituiertes $C_2$-$C_4$-Alkanoyloxymethyl darstellen, $R^1$ Carboxy, Carbamoyl oder $C_1$-$C_4$-Alkoxycarbonyl darstellt, $R^2$ und $R^3$ Wasserstoff darstellen und A an der 4-Position ist und $C_1$-$C_4$-Alkylen darstellt ; oder eines pharmazeutisch annehmbaren Salzes davon.

4. Verfahren nach Anspruch 1 zur Herstellung einer Verbindung der Formel III

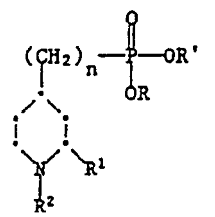

(III)

worin n die ganze Zahl 1, 2 oder 3 darstellt ; R und R' unabhängig Wasserstoff, $C_1$-$C_4$-Alkyl, Benzyl, $C_2$-$C_4$-Alkanoyloxymethyl oder am Oxymethyl durch $C_1$-$C_4$-Alkyl substituiertes $C_2$-$C_4$-Alkanoyloxymethyl darstellen, $R^1$ Carboxy, $C_1$-$C_4$-Alkoxycarbonyl oder Carbamoyl darstellt und $R^2$ Wasserstoff, $C_1$-$C_4$-Alkyl, $C_2$-$C_4$-Alkanoyl oder Benzoyl darstellt : oder eines pharmazeutisch annehmbaren Salzes davon.

5. Verfahren nach Anspruch 1 zur Herstellung einer Verbindung der Formel III nach Anspruch 4, worin n die ganze Zahl 1, 2 oder 3 ist ; R und R' beide Wasserstoff oder $C_2$-$C_4$-Alkanoyloxymethyl sind ; oder eines von R und R' Wasserstoff ist und das andere von R und R' $C_1$-$C_4$-Alkyl, Benzyl, $C_2$-$C_4$-Alkanoyloxymethyl oder am Oxymethyl durch $C_1$-$C_4$-Alkyl substituiertes $C_2$-$C_4$-Alkanoyloxymethyl ist, $R^1$ Carboxy oder $C_1$-$C_4$-Alkoxycarbonyl darstellt und $R^2$ Wasserstoff ist ; oder eines pharmazeutisch annehmbaren Salzes davon.

6. Verfahren nach Anspruch 1 zur Herstellung einer Verbindung der Formel III nach Anspruch 4, worin n die ganze Zahl 1, 2 oder 3 ist ; R und R' Wasserstoff sind ; $R^1$ Carboxy oder $C_1$-$C_4$-Alkoxycarbonyl ist und $R^2$ Wasserstoff darstellt ; oder eines pharmazeutisch annehmbaren Salzes davon.

7. Verfahren nach Anspruch 1 zur Herstellung von cis-4-Phosphonomethyl-2-piperidincarbonsäure oder eines pharmazeutisch annehmbaren Salzes davon.

8. Verfahren nach Anspruch 1 zur Herstellung von cis-4-(3-Phosphonopropyl)-2-piperidincarbonsäure oder eines pharmazeutisch annehmbaren Salzes davon.

9. Verfahren nach Anspruch 1 zur Herstellung von cis-4-Phosphonomethyl-2-piperidincarbonsäureethylester oder eines pharmazeutisch annehmbaren Salzes davon.

10. Verfahren nach Anspruch 1 zur Herstellung einer Verbindung der Formel IV

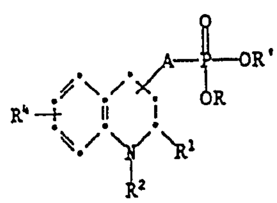

(IV)

oder eines Perhydro-Derivats davon, worin R und R' unabhängig Wasserstoff, $C_1$-$C_4$-Alkyl, Benzyl, am Phenyl durch Halogen, $C_1$-$C_4$-Alkyl oder $C_1$-$C_4$-Alkoxy substituiertes Benzyl ; $C_2$-$C_4$-Alkanoyloxymethyl oder am Oxymethyl durch $C_1$-$C_4$-Alkyl oder Cycloalkyl substituiertes $C_2$-$C_4$-Alkanoyloxymethyl darstellen, $R^1$ Carboxy, $C_1$-$C_4$-Alkoxycarbonyl, Carbamoyl oder N-Mono- oder N,N-Di-$C_1$-$C_4$-alkylcarbamyl ist, $R^2$ Wasserstoff, $C_1$-$C_4$-Alkyl, Aryl-$C_1$-$C_4$-alkyl, $C_2$-$C_7$-Alkanoyl, Benzoyl, $C_1$-$C_4$-Alkoxycarbonyl, Phenylacetyl, 3-Phenylpropionyl oder α-Amino-$C_2$-$C_4$-alkanoyl ist, $R^4$ Wasserstoff, niederes Alkyl, niederes Alkoxy, Halogen oder Trifluormethyl ist und A für $C_1$-$C_4$-Alkylen steht ; oder eines pharmazeutisch annehmbaren Salzes davon.

11. Verfahren nach Anspruch 1 zur Herstellung einer Verbindung der Formel V

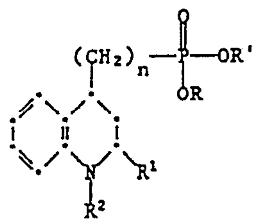

(V)

oder eines Perhydrochinolin-Derivates davon, worin n die ganze Zahl 1, 2 oder 3 ist; R und R′ unabhängig Wasserstoff, $C_1$-$C_4$-Alkyl, Benzyl, $C_2$-$C_4$-Alkanoyloxymethyl oder am Oxymethyl durch $C_1$-$C_4$-Alkyl substituiertes $C_2$-$C_4$-Alkanoyloxymethyl ist, $R^1$ Carboxy, $C_1$-$C_4$-Alkoxycarbonyl oder Carbamoyl ist, $R^2$ Wasserstoff, $C_1$-$C_4$-Alkyl, $C_2$-$C_4$-Alkanoyl, Benzoyl oder durch $C_1$-$C_4$-Alkyl, $C_1$-$C_4$-Alkoxy, Halogen oder Trifluormethyl substituiertes Benzoyl ist; oder eines pharmazeutisch annehmbaren Salzes davon.

12. Verfahren nach Anspruch 1 zur Herstellung einer verbindung der Formel V nach Anspruch 11, worin n die ganze Zahl 1, 2 oder 3 darstellt; R und R′ Wasserstoff darstellen; $R^1$ Carboxy oder $C_1$-$C_4$-Alkoxycarbonyl ist und $R^2$ Wasserstoff darstellt; oder eines pharmazeutisch annehmbaren Salzes davon.

13. Verfahren nach Anspruch 1 zur Herstellung von cis-4-Phosphonomethyl-1,2,3,4-tetrahydrochinolin-2-carbonsäure oder eines pharmazeutisch annehmbaren Salzes davon ist.

14. Verfahren nach Anspruch 1 zur Herstellung einer verbindung der Formel VI

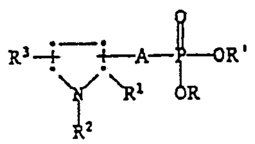

(VI)

oder einer verbindung der Formel VI mit einer Doppelbindung zwischen C-3 und C-4 des Pyrrolidinylringes, worin R und R′ unabhängig für Wasserstoff, $C_1$-$C_4$-Alkyl, Benzyl, am Phenyl durch Halogen, $C_1$-$C_4$-Alkyl oder $C_1$-$C_4$-Alkoxy substituiertes Benzyl; $C_2$-$C_4$-Alkanoyloxymethyl oder am Oxymethyl durch $C_1$-$C_4$-Alkyl oder Cycloalkyl substituiertes $C_2$-$C_4$-Alkanoyloxymethyl ist, $R^1$ Carboxy, $C_1$-$C_4$-Alkoxycarbonyl, Carbamoyl oder N-Mono- oder N, N-Di-$C_1$-$C_4$-alkylcarbamoyl ist, $R^2$ Wasserstoff, $C_1$-$C_4$-Alkyl, $C_2$-$C_7$-Alkanoyl, Benzoyl, $C_1$-$C_4$-Alkoxycarbonyl, Phenylacetyl, 3-Phenylpropionyl oder α-Amino-$C_2$-$C_4$-alkanoyl ist, $R^3$ Wasserstoff, $C_1$-$C_4$-Alkyl oder Aryl-$C_1$-$C_4$-alkyl ist und A $C_1$-$C_4$-Alkylen darstellt; oder eines pharmazeutisch annehmbaren Salzes davon.

15. Verfahren nach Anspruch 1 zur Herstellung einer verbindung der Formel VI nach Anspruch 14, worin die Phosphono-tragende Gruppe an der 3- oder 4-Position gebunden ist, R und R′ unabhängig Wasserstoff oder $C_2$-$C_4$-Alkanoyloxymethyl sind, $R^1$ Carboxy, $C_1$-$C_4$-Alkorycarbonyl oder Carbamoyl ist, $R^2$ und $R^3$ Wasserstoff sind und A $C_1$-$C_3$-Alkylen ist; oder eines pharmazeutisch annehmbaren Salzes davon.

16. Verfahren nach Anspruch 1 zur Herstellung von 4-Phosphonomethylpyrrolidin-2-carbonsäure oder eines pharmazeutisch annehmbaren Salzes davon.

17. Verfahren zur Herstellung pharmazeutischer Zusammensetzungen, worin eine nach irgendeinem der Ansprüche 1 bis 16 erhältliche verbindung mit einem oder mit mehreren pharmazeutisch annehmbaren Trägern vermischt wird.

## Revendications

### Revendications pour les Etats Contractants BE, CH, DE, FR, GB, IT, LI, LU, NL, SE

1. Composé de formule I,

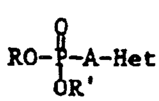

(I)

où R et R′ représentent, indépendamment l'un de l'autre, l'hydrogène, ou un groupe alkyle en $C_1$ -$C_4$, benzyle, benzyle substitué sur le radical phényle par un halogène, un radical alkyle en $C_1$-$C_4$ ou un radical alcoxy en

$C_1$-$C_4$, alcanoyl($C_2$-$C_7$)oxyméthyle ou alcanoyl($C_2$-$C_7$)oxyméthyle substitué sur le radical oxyméthyle par un radical alkyle en $C_1$-$C_4$ ou cycloalkyle en $C_3$-$C_8$, A représente un groupe alkylène en $C_1$-$C_4$ et Het représente un groupe 2-$R^1$-pyrrolidinyle, 2-$R^1$-2,5-dihydropyrrolyle, 2-$R^1$-1,2,3,6-tétrahydropyridinyle, 2-$R^1$-1,2,5,6-tétrahydropyridinyle, 2-$R^1$-pipéridinyle, 2-$R^1$-tétrahydroquinoléyle, 2-$R^1$-perhydroquinolfyle, 2-$R^1$-carboxy-2,3-dihydroindolyle ou 2-$R^1$-perhydroindolyle, qui peut aussi être N-substitué par un radical alkyle en $C_1$-$C_4$, phénylalkyle en $C_1$-$C_4$, alcanoyle en $C_2$-$C_7$, benzoyle, alcoxy($C_1$-$C_4$)carbonyle, benzyloxycarbonyle, phénylacétyle, 3-phénylpropionyle ou α-aminoalkyle en $C_2$-$C_4$, C-substitué sur l'hétérocycle à 5 ou 6 chaînons par un radical alkyle en $C_1$-$C_4$ ou phénylalkyle en $C_1$-$C_4$ et/ou, s'il est présent, sur le cycle benzo ou cyclohexano par un radical alkyle en $C_1$-$C_4$, alcoxy en $C_1$-$C_4$, halogéno ou trifluorométhyle, ou un sel de ce composé.

2. Composé selon la revendication 1, caractérisé en ce qu'il répond à la formule II

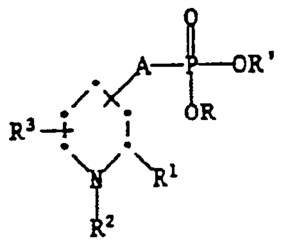

(II)

ou composé de formule II avec une double liaison présente entre les atomes de carbone 3 et 4 ou entre les atomes de carbone 4 et 5 du cycle pipéridinyle, dans lesquels la chaîne portant le groupe phosphono est fixée sur la position 3, 4 ou 5 du cycle pipéridinyle ou tétrahydropyridinyle, et où R et R′ représentent, indépendamment l'un de l'autre, l'hydrogène ou un radical alkyle en $C_1$-$C_4$, benzyle, alcanoyl($C_2$-$C_4$)oxyméthyle ou alcanoyl($C_2$-$C_4$)oxyméthyle substitué sur le groupe oxyméthyle par des radicaux alkyle en $C_1$-$C_4$, cyclohexyle ou cyclopentyle, $R^1$ représente un radical carboxy, carbamoyle, N-mono- ou N,N-di-alkyl($C_1$-$C_4$)carbamoyle ou alcoxy($C_1$-$C_4$)carbonyle, $R^2$ et $R^3$ représentent l'hydrogène ou un radical alkyle en $C_1$-$C_4$ et A représente un radical alkylène en $C_1$-$C_4$ ; ou un sel pharmaceutiquement acceptable de ce composé.

3. Composé de formule II selon la revendication 2, caractérisé en ce que R et R′, indépendamment l'un de l'autre, représentent l'hydrogène ou un radical alcanoyl ($C_2$-$C_4$)oxyméthyle ou alcanoyl($C_2$-$C_4$)oxyméthyle substitué sur le groupe oxyméthyle par un radical alkyle en $C_1$-$C_4$, $R^1$ représente un radical carboxy, carbamoyle ou alcoxy ($C_1$-$C_4$)carbonyle, $R^2$ et $R^3$ représentent l'hydrogène et A est sur la position 4 et représente un radical alkylène en $C_1$-$C_4$ ; ou un sel pharmaceutiquement acceptable de ce composé.

4. Composé selon la revendication 2, caractérisé en ce qu'il répond à la formule III

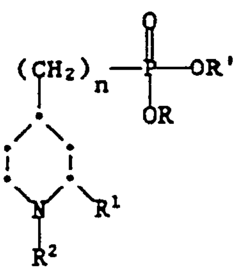

(III)

où n représente l'entier 1, 2 ou 3 ; R et R′, indépendamment l'un de l'autre, représentent l'hydrogène ou un radical alkyle en $C_1$-$C_4$, benzyle, alcanoyl($C_2$-$C_4$)oxyméthyle ou alcanoyl($C_2$-$C_4$)oxyméthyle substitué sur le groupe oxyméthyle par un radical alkyle en $C_1$-$C_4$, $R^1$ représente un radical carboxy, alcoxy($C_1$-$C_4$)carbonyle ou carbamoyle et $R^2$ représente l'hydrogène ou un radical alkyle en $C_1$-$C_4$, alcanoyle en $C_2$-$C_4$ ou benzoyle ; ou un sel pharmaceutiquement acceptable de ce composé ayant un groupement fonctionnel salifiable.

5. Composé de formule III selon la revendication 4, caractérisé en ce que n représente l'entier 1, 2 ou 3 ; R et R′ représentent chacun l'hydrogène ou un radical alcanoyl($C_2$-$C_4$)oxyméthyle ; ou l'un des groupes R et R′ représente l'hydrogène et l'autre de ces groupes R et R′ représente un radical alkyle en $C_1$-$C_4$, benzyle, alcanoyl ($C_2$-$C_4$ )oxyméthyle ou alcanoyl($C_2$-$C_4$)oxyméthyle substitué sur le groupe oxyméthyle par un radical alkyle en $C_1$-$C_4$, $R^1$ représente un radical carboxy ou alcoxy($C_1$-$C_4$)carbonyle et $R^2$ représente l'hydrogène ; ou un sel pharmaceutiquement acceptable de ce composé.

6. Composé de formule III selon la revendication 4, caractérisé en ce que n représente l'entier 1, 2 ou 3 ; R et R′ représentent l'hydrogène, $R^1$ représente un radical carboxy ou alcoxy($C_1$-$C_4$)carbonyle et $R^2$ représente

l'hydrogène ; ou un sel pharmaceutiquement acceptable de ce composé.

7. Composé selon la revendication 6, caractérisé en ce qu'il est l'acide cis-4-phosphonométhyl-2-pipéridinecarboxylique ou un sel pharmaceutiquement acceptable de celui-ci.

8. Composé selon la revendication 6, caractérisé en ce qu'il est l'acide cis-4-(3-phosphonopropyl)-2-pipéridine-carboxylique ou un sel pharmaceutiquement acceptable de celui-ci.

9. Composé selon la revendication 6, caractérisé en ce qu'il est l'ester éthylique de l'acide cis-4-phosphonométhyl-2-pipéridinecarboxylique ou un sel pharmaceutiquement acceptable de celui-ci.

10. Composé selon la revendication 1, caractérisé en ce qu'il répond à la formule IV

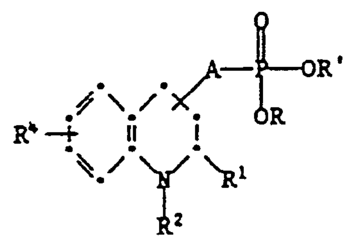

(IV)

ou un dérivé perhydro de celle-ci, où R et R', indépendamment l'un de l'autre, représentent l'hydrogène ou un radical alkyle en $C_1$-$C_4$, benzyle, benzyle substitué sur le groupe phényle par un halogène ou un radical alkyle en $C_1$-$C_4$ ou alcoxy en $C_1$-$C_4$ ; alcanoyl ($C_2$-$C_4$)oxyméthyle ou alcanoyl ($C_2$-$C_4$)oxyméthyle substitué sur le groupe oxyméthyle par un radical alkyle en $C_1$-$C_4$ ou cycloalkyle, $R^1$ représente un radical carboxy, alcoxy ($C_1$-$C_4$)carbonyle, carbamoyle ou N-mono- ou N,N-di-alkyl ($C_1$-$C_4$)carbamoyle, $R^2$ représente l'hydrogène ou un radical alkyle en $C_1$-$C_4$, arylalkyle en $C_1$-$C_4$, alcanoyle en $C_2$-$C_7$, benzoyle, alcoxy ($C_1$-$C_4$)carbonyle, phénylacétyle, 3-phénylpropionyle ou α-amino-alcanoyle en $C_2$-$C_4$, $R^4$ représente l'hydrogène ou un radical alkyle inférieur, alcoxy inférieur, halogène ou trifluorométhyle, et A représente un radical alkylène en $C_1$-$C_4$ ; ou un sel pharmaceutiquement acceptable de ce composé.

11. Composé selon la revendication 10, caractérisé en ce qu'il répond à la formule V

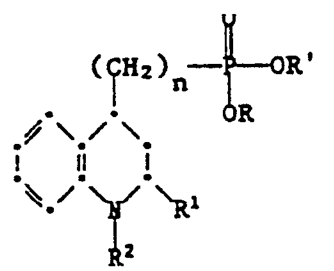

(V)

ou un dérivé de perhydroquinoléine de celle-ci, ou n représente l'entier 1, 2 ou 3 ; R et R', indépendamment l'un de l'autre, représentent l'hydrogène ou un radical alkyle en $C_1$-$C_4$, benzyle, alcanoyl ($C_2$-$C_4$)oxyméthyle ou alcanoyl($C_2$-$C_4$) oxyméthyle substitué sur le groupe oxyméthyle par un radical alkyle en $C_1$-$C_4$, $R^1$ représente un radical carboxy, alcoxy($C_1$-$C_4$)carbonyle ou carbamoyle, $R^2$ représente l'hydrogène ou un radical alkyle en $C_1$-$C_4$, alcanoyle en $C_2$-$C_4$, benzoyle ou benzoyle substitué par un groupe alkyle en $C_1$-$C_4$, alcoxy en $C_1$-$C_4$, halogène ou trifluorométhyle ; ou un sel pharmaceutiquement acceptable de ce composé ayant un groupement fonctionnel salifiable.

12. Composé de formule V selon la revendication 11, caractérisé en ce que n représente l'entier 1, 2 ou 3 ; R et R' représentent l'hydrogène, $R^1$ représente un radical carboxy ou alcoxy($C_1$-$C_4$)carbonyle et $R^2$ représente l'hydrogène ; ou un sel pharmaceutiquement acceptable de ce composé.

13. Composé selon la revendication 12, caractérisé en ce qu'il est l'acide cis-4-phosphonométhyl-1,2,3,4-tétrahydroquinoléine-2-carboxylique ou un sel pharmaceutiquement acceptable de celui-ci.

14. Composé selon la revendication 1, caractérisé en ce qu'il répond à la formule VI

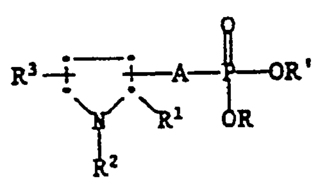

(VI)

ou un composé de formule VI ayant une double liaison entre les atomes de carbone 3 et 4 du cycle pyrrolidinyle, dans lesquels R et R', indépendamment l'un de l'autre, représentent l'hydrogène ou un radical alkyle en $C_1$-$C_4$, benzyle, benzyle substitué sur le groupe phényle par un halogène ou un groupe alkyle en $C_1$-$C_4$ ou alcoxy en $C_1$-$C_4$ ; alcanoyl($C_2$-$C_4$)oxyméthyle ou alcanoyl($C_2$-$C_4$)oxyméthyle substitué sur le groupe oxyméthyle par un radical alkyle en $C_1$-$C_4$ ou cycloalkyle, $R^1$ représente un radical carboxy, alcoxy($C_1$-$C_4$)carbonyle, carbamoyle ou N-mono- ou N,N-di-alkyl($C_1$-$C_4$) carbamoyle, $R^2$ représente l'hydrogène ou un radical alkyle en $C_1$-$C_4$, alcanoyle en $C_2$-$C_7$, benzoyle, alcoxy($C_1$-$C_4$) carbonyle, phénylacétyle, 3-phénylpropionyle ou α-aminoalcanoyle en $C_2$-$C_4$, $R^3$ représente l'hydrogène ou un radical alkyle en $C_1$-$C_4$ ou arylalkyle en $C_1$-$C_4$ et A représente un radical alkylène en $C_1$-$C_4$ ; ou un sel de ce composé.

15. Composé de formule VI selon la revendication 14, caractérisé en ce que le groupe portant le radical phosphono est fixé sur la position 3 ou 4, R et R', indépendamment l'un de l'autre, représentent l'hydrogène ou un radical alcanoyl($C_2$-$C_4$)oxyméthyle, $R^1$ représente un radical carboxy, alcoxy($C_1$-$C_4$)carbonyle ou carbamoyle ; $R^2$ et $R^3$ représentent l'hydrogène et A représente un radical alkylène en $C_1$-$C_3$ ; ou un sel pharmaceutiquement acceptable de ce composé.

16. Composé selon la revendication 15, caractérisé en ce qu'il est l'acide 4-phosphonométhylpyrrolidine-2-carboxylique ou un sel pharmaceutiquement acceptable de celui-ci

17. Composé selon l'une quelconque des revendications 2 à 5, 7 à 9, 11 à 13, 15 et 16 pour utilisation dans le traitement thérapeutique du corps humain ou animal.

18. Composé selon l'une quelconque des revendications 1, 6, 10 et 14 pour utilisation dans le traitement thérapeutique du corps humain ou animal.

19. Composition pharmaceutique, caractérisée en ce qu' elle comprend un composé selon l'une quelconque des revendications 2 à 5, 7 à 9, 11 à 13 et 17 en mélange ou en combinaison avec un ou plusieurs véhicules pharmaceutiquement appropriés.

20. Composition pharmaceutique, caractérisée en ce qu' elle comprend un composé selon l'une quelconque des revendications 1, 6, 10, 14 et 18 en mélange ou en combinaison avec un ou plusieurs véhicules pharmaceutiquement appropriés.

21. Composé selon l'une quelconque des revendications 1 à 16 en tant qu'antagoniste du récepteur d'amino acide excitatoire sensible au N-méthyl-D-aspartate (NMDA).

22. Utilisation d'un composé selon l'une quelconque des revendications 1 à 16 pour la préparation d'une composition pharmaceutique.

23. Utilisation d'un composé selon l'une quelconque des revendications 1 à 16 pour la préparation d'une composition pharmaceutique pour le traitement des maladies induites par le blocage du récepteur d'amino acide excitatoire sensible au N-méthyl-D-aspartate (NMDA).

24. Procédé pour la préparation d'un composé de formule I selon la revendication 1, ou d'un sel de celui-ci, caractérisé en ce qu'il consiste en

a) la réduction d'un composé de formule VII

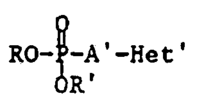

(VII)

ledit composé de formule VII étant identique par ailleurs à un composé de formule I, mais ayant une ou plusieurs doubles liaisons présentes dans l'hétérocycle à cinq ou six chaînons Het' et/ou dans A' qui représente A ou un radical alcénylène inférieur ou alcanylidène inférieur avec la double liaison fixée sur l'hétérocycle ; ou

b) la condensation d'un composé de formule VIII

X-A-Het (VIII)

où Het et A sont tels que définis pour la formule I et X représente un radical hydroxy estérifié réactif, avec un composé capable d'introduire la fraction acide phosphonique, ayant l'une des formules IX ou X,

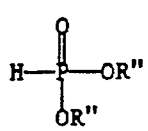

(IX) P(R''')$_3$ (X)

où R" représente un radical alkyle inférieur et R''' représente un halogène ou un radical alcoxy inférieur et, si nécessaire, conversion du dérivé d'acide phosphonique résultant en l'acide phosphonique ou en un autre dérivé de type ester de celui-ci ; ou

c) la conversion en $R^1$ d'un substituant autre que $R^1$ sur la position 2 de l'hétérocycle dans un composé par ailleurs identique à un composé de l'invention ; et mise en oeuvre desdits procédés tandis que, si nécessaire, on protège temporairement tout(ou tous) groupe(s) réactif(s) interférant dans ces procédés, et ensuite libération du composé résultant de l'invention ; et, si on le souhaite, conversion d'un composé résultant selon l'invention en un autre composé de l'invention, et/ou, si on le souhaite, conversion d'un composé résultant libre en un sel ou d'un sel résultant en le composé libre ou en un autre sel ; et/ou séparation d'un mélange d'isomères ou de racémiques obtenus en les isomères individuels ou les racémiques; et/ou, si on le souhaite, dédoublement d'un racémique obtenu en ses inverses optiques.

## Revendications pour l'Etat contractant AT

1. Composé de formule I

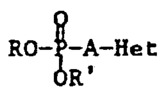

(I)

où R et R' représentent, indépendamment l'un de l'autre, l'hydrogène, ou un groupe alkyle en $C_1$-$C_4$, benzyle, benzyle substitué sur le radical phényle par un halogène, un radical alkyle en $C_1$-$C_4$ ou un radical alcoxy en $C_1$-$C_4$, alcanoyl($C_2$-$C_7$)oxyméthyle ou alcanoyl($C_2$-$C_7$)oxyméthyle substitué sur le radical oxyméthyle par un radical alkyle en $C_1$-$C_4$ ou cycloalkyle en $C_3$-$C_8$, A représente un groupe alkylène en $C_1$-$C_4$ et Het représente un groupe 2-$R^1$-pyrrolidinyle, 2-$R^1$-2,5-dihydro-pyrrolyle, 2-$R^1$-1,2,3,6-tétrahydropyridinyle, 2-$R^1$-1,2,5,6-tétrahydrcpyridinyle, 2-$R^1$-pipéridinyle, 2-$R^1$-tétrahydroquinoléyle, 2-$R^1$-perhydroquinoléyle, 2-$R^1$-carboxy-2,3-dihydroindolyle ou 2-$R^1$-perhycroindolyle, qui peut aussi être N-substitué par un radical alkyle en $C_1$-$C_4$, phénylalkyle en $C_1$-$C_4$, alcanoyle en $C_2$-$C_7$, benzoyle, alcoxy($C_1$-$C_4$)carbonyle, benzyloxycarbonyle, phénylacétyle, 3-phénylpropionyle ou $\alpha$-aminoalkyle en $C_2$-$C_4$, C-substitué sur l'hétérocycle à 5 ou 6 chaînons par un radical alkyle en $C_1$-$C_4$ ou phénylalkyle en $C_1$-$C_4$ et/ou, s'il est présent, sur le cycle benzo ou cyclohexano par un radical alkyle en $C_1$-$C_4$, alcoxy en $C_1$-$C_4$, halogéno ou trifluorométhyle, ou un sel de ce composé, caractérisé en ce qu'il consiste en

a) la réduction d'un composé de formule VII

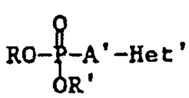

(VII)

ledit composé de formule VII étant identique par ailleurs à un composé de formule I, mais ayant une ou plusieurs doubles liaisons présentes dans l'hétérocycle à cinq ou six chaînons Het' et/ou dans A' qui représente A ou un radical alcénylène inférieur ou alcanylidène inférieur avec la double liaison fixée sur l'hétérocycle ; ou

b) la condensation d'un composé de formule VIII

X-A-Het (VIII)

où Het et A sont tels que définis pour la formule I et X représente un radical hydroxy estérifié réactif, avec un composé capable d'introduire la fraction acide phosphonique, ayant l'une des formules IX ou X,

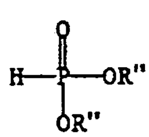

(IX) $P(R''')_3$ (X)

où R" représente un radical alkyle inférieur et R''' représente un halogène ou un radical alcoxy inférieur et,

si nécessaire, conversion du dérivé d'acide phosphonique résultant en l'acide phosphonique ou en un autre dérivé de type ester de celui-ci ; ou

c) la conversion en $R^1$ d'un substituant autre que $R^1$ sur la position 2 de l'hétérocycle dans un composé par ailleurs identique à un composé de l'invention ; et mise en oeuvre desdits procédés tandis que, si nécessaire, on protège temporairement tout(ou tous) groupe(s) réactif(s) interférant dans ces procédés, et ensuite libération du composé résultant de l'invention ; et, si on le souhaite, conversion d'un composé résultant selon l'invention en un autre composé de l'invention, et/ou, si on le souhaite, conversion d'un composé résultant libre en un sel ou d'un sel résultant en le composé libre ou en un autre sel ; et/ou séparation d'un mélange d'isomères ou de racémiques obtenus en les isomères individuels ou les racémiques; et/ou, si on le souhaite, dédoublement d'un racémique obtenu en ses inverses optiques.

2. Procédé selon la revendication 1 pour la préparation d'un composé de formule II

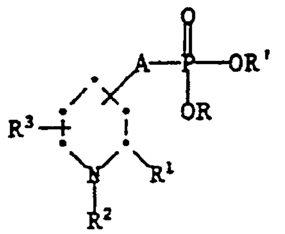

(II)

ou un composé de formule II avec une double liaison présente entre les atomes de carbone 3 et 4 ou entre les atomes de carbone 4 et 5 du cycle pipéridinyle, dans lesquels la chaîne portant le groupe phosphono est fixée sur la position 3, 4 ou 5 du cycle pipéridinyle ou tétrahydropyridinyle, et où R et R′ représentent, indépendamment l'un de l'autre, l'hydrogène ou un radical alkyle en $C_1$-$C_4$, benzyle, alcanoyl($C_2$-$C_4$)oxyméthyle ou alcanoyl($C_2$-$C_4$)oxyméthyle substitué sur le groupe oxyméthyle par des radicaux alkyle en $C_1$-$C_4$, cyclohexyle ou cyclopentyle, $R^1$ représente un radical carboxy, carbamoyle, N-mono- ou N,N-di-alkyl($C_1$-$C_4$)carbamoyle ou alcoxy($C_1$-$C_4$)carbonyle, $R^2$ et $R^3$ représentent l'hydrogène ou un radical alkyle en $C_1$-$C_4$ et A représente un radical alkylène en $C_1$-$C_4$ ; ou un sel pharmaceutiquement acceptable de ce composé.

3. Procédé selon la revendication 1 pour la préparation d'un composé de formule II selon la revendication 2, où R et R′, indépendamment l'un de l'autre, représentent l'hydrogène ou un radical alcanoyl($C_2$-$C_4$)oxyméthyle ou alcanoyl($C_2$-$C_4$) oxyméthyle substitué sur le groupe oxyméthyle par un radical alkyle en $C_1$-$C_4$, $R^1$ représente un radical carboxy, carbamoyle ou alcoxy($C_1$-$C_4$)carbonyle, $R^2$ et $R^3$ représentent l'hydrogène et A est sur la position 4 et représente un radical alkylène en $C_1$-$C_4$ ; ou d' un sel pharmaceutiquement acceptable de ce composé.

4. Procédé selon la revendication 1 pour la préparation d'un composé de formule III

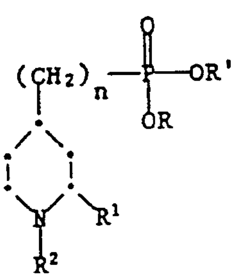

(III)

où n représente l'entier 1, 2 ou 3 ; R et R′, indépendamment l'un de l'autre, représentent l'hydrogène ou un radical alkyle en $C_1$-$C_4$, benzyle, alcanoyl ($C_2$-$C_4$)oxyméthyle ou alcanoyl ($C_2$-$C_4$)oxyméthyle substitué sur le groupe oxyméthyle par un radical alkyle en $C_1$-$C_4$, $R^1$ représente un radical carboxy, alcoxy ($C_1$-$C_4$)carbonyle ou carbamoyle et $R^2$ représente l'hydrogène ou un radical alkyle en $C_1$-$C_4$, alcanoyle en $C_2$-$C_4$ ou benzoyle ; ou d'un sel pharmaceutiquement acceptable de ce composé.

5. Procédé selon la revendication 1 pour la préparation d'un composé de formule III selon la revendication 4, où n représente l'entier 1, 2 ou 3 ; R et R′ représentent chacun l'hydrogène ou un radical alcanoyl ($C_2$-$C_4$)oxyméthyle ; ou l'un des groupes R et R′ représente l'hydrogène et l'autre de ces groupes R et R′ représente un radical alkyle en $C_1$-$C_4$, benzyle, alcanoyl ($C_2$-$C_4$)oxyméthyle ou alcanoyl ($C_2$-$C_4$)oxyméthyle substitué sur le groupe oxyméthyle par un radical alkyle en $C_1$-$C_4$, $R^1$ représente un radical carboxy ou alcoxy ($C_1$-$C_4$)carbonyle et $R^2$ représente l'hydrogène ; ou d'un sel pharmaceutiquement acceptable de ce composé.

6. Procédé selon la revendication 1 pour la préparation d'un composé de formule III selon la revendication 4, où n représente l'entier 1, 2 ou 3 ; R et R' représentent l'hydrogène, $R^1$ représente un radical carboxy ou alcoxy ($C_1$-$C_4$)carbonyle et $R^2$ représente l'hydrogène ; ou d'un sel pharmaceutiquement acceptable de ce composé.

7. Procédé selon la revendication 1 pour la préparation de l'acide cis-4-phosphonométhyl-2-pipéridinecarboxylique ou d'un sel pharmaceutiquement acceptable de ce composé.

8. procédé selon la revendication 1 pour la préparation de l'acide cis-4-(3-phosphonopropyl)-2-pipéridinecarboxylique ou d'un sel pharmaceutiquement acceptable de ce composé.

9. procédé selon la revendication 1 pour la préparation de l'ester éthylique de l'acide cis-4-phosphonométhyl-2-pipéridinecarboxylique ou d'un sel pharmaceutiquement acceptable de ce composé.

10. procédé selon la revendication 1 pour la préparation d'un composé de formule IV

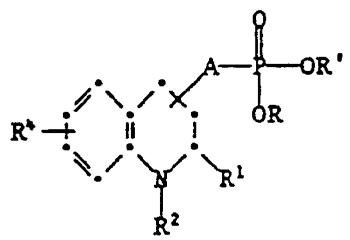

(IV)

ou d'un dérivé perhydro de celle-ci, où R et R', indépendamment l'un de l'autre, représentent l'hydrogène ou un radical alkyle en $C_1$-$C_4$, benzyle, benzyle substitué sur le groupe phényle par un halogène ou un radical alkyle en $C_1$-$C_4$ ou alcoxy en $C_1$-$C_4$ ; alcanoyl($C_2$-$C_4$)oxyméthyle ou alcanoyl($C_2$-$C_4$)oxyméthyle substitué sur le groupe oxyméthyle par un radical alkyle en $C_1$-$C_4$ ou cycloalkyle, $R^1$ représente un radical carboxy, alcoxy($C_1$-$C_4$)carbonyle, carbamoyle ou N-mono- ou N,N-di-alkyl($C_1$-$C_4$)carbamoyle, $R^2$ représente l'hydrogène ou un radical alkyle en $C_1$-$C_4$, arylalkyle en $C_1$-$C_4$, alcanoyle en $C_2$-$C_7$, benzoyle, alcoxy ($C_1$-$C_4$)carbonyle, phénylacétyle, 3-phénylpropionyle ou α-amino-alcanoyle en $C_2$-$C_4$, $R^4$ représente l'hydrogène ou un radical alkyle inférieur, alcoxy inférieur, halogène ou trifluorométhyle, et A représente un radical alkylène en $C_1$-$C_4$ ; ou d'un sel pharmaceutiquement acceptable de ce composé.

11. Procédé selon la revendication 1 pour la préparation d'un composé de formule V

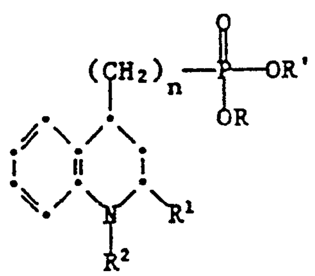

(V)

ou d'un dérivé de perbydroquinoléine de celle-ci, où n représente l'entier 1, 2 ou 3 ; R et R', indépendamment l'un de l'autre, représentent l'hydrogène ou un radical alkyle en $C_1$-$C_4$, benzyle, alcanoyl($C_2$-$C_4$)oxyméthyle ou alcanoyl ($C_2$-$C_4$) oxymétbyle substitué sur le groupe oxyméthyle par un radical alkyle en $C_1$-$C_4$, $R^1$ représente un radical carboxy, alcoxy($C_1$-$C_4$ )carbonyle ou carbamoyle, $R^2$ représente l'hydrogène ou un radical alkyle en $C_1$-$C_4$, alcanoyle en $C_2$-$C_4$, benzoyle ou benzoyle substitué par un groupe alkyle en $C_1$-$C_4$, alcoxy en $C_1$-$C_4$, halogène ou trifluorométhyle ; ou d'un sel pharmaceutiquement acceptable de ce composé.

12. Procédé selon la revendication 1 pour la préparation d'un composé de formule V selon la revendication 11, où n représente l'entier 1, 2 ou 3 ; R et R' représentent l'hydrogène, $R^1$ représente un radical carboxy ou alcoxy ($C_1$-$C_4$)carbonyle et $R^2$ représente l'hydrogène ; ou d'un sel pharmaceutiquement acceptable de ce composé.

13. Procédé selon la revendication 1 pour la préparation d'acide cis-4-phosphonométhyl-1,2,3,4-tétrabydroquinoléine-2-carboxylique ou d'un sel pharmaceutiquement acceptable de ce composé.

14. Procédé selon la revendication 1 pour la préparation d'un composé de formule VI

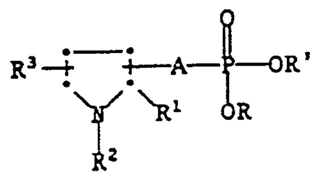

(VI)

ou d'un composé de formule VI ayant une double liaison entre les atomes de carbone 3 et 4 du cycle pyrrolidinyle, dans lesquels R et R', indépendamment l'un de l'autre, représentent l'hydrogène ou un radical alkyle en $C_1$-$C_4$, benzyle, benzyle substitué sur le groupe phényle par un halogène ou un groupe alkyle en $C_1$-$C_4$ ou alcoxy en $C_1$-$C_4$ ; alcanoyl($C_2$-$C_4$)oxyméthyle ou alcanoyl($C_2$-$C_4$)oxyméthyle substitué sur le groupe oxyméthyle par un radical alkyle en $C_1$-$C_4$ ou cycloalkyle, $R^1$ représente un radical carboxy, alcoxy($C_1$-$C_4$)carbonyle, carbamoyle ou N-mono- ou N,N-dialykl($C_1$-$C_4$)carbamoyle, $R^2$ représente l'hydrogène ou un radical alkyle en $C_1$-$C_4$, alcanoyle en $C_2$-$C_7$, benzoyle, alcoxy($C_1$-$C_4$)carbonyle, phénylacétyle, 3-phénylpropionyle ou $\alpha$-amino-alcanoyle en $C_2$-$C_4$, $R^3$ représente l'hydrogène ou un radical alkyle en $C_1$-$C_4$ ou arylalkyle en $C_1$-$C_4$ et A représente un radical alkylène en $C_1$-$C_4$ ; ou d'un sel pharmaceutiquement acceptable de ce composé.

15. Procédé selon la revendication 1 pour la préparation d'un composé de formule VI selon la revendication 14, où le groupe portant le radical phosphono est fixé sur la position 3 ou 4, R et R', indépendamment l'un de l'autre, représentent l'hydrogène ou un radical alcanoyl($C_2$-$C_4$)oxyméthyle, $R^1$ représente un radical carboxy, alcoxy($C_1$-$C_4$) carbonyle ou carbamoyle, $R^2$ et $R^3$ représentent l'hydrogène et A représente un radical alkylène en $C_1$-$C_3$ ; ou d'un sel pharmaceutiquement acceptable de ce composé.

16. Procédé selon la revendication 1 pour la préparation de l'acide 4-phosphonométhylpyrrolidine-2-carboxylique ou d'un sel pharmaceutiquement acceptable de ce composé.

17. Procédé pour la préparation d'une composition pharmaceutique, caractérisé en ce qu'on mélange un composé qui peut être obtenu selon l'une quelconque des revendications 1 à 16 avec un ou plusieurs véhicules pharmaceutiquement acceptables.